# EUROPEAN PATENT SPECIFICATION

(11) **EP 1 304 103 B1**
(45) Date of publication and mention of the grant of the patent: **31.12.2008**
(21) Application number: 01402737.9
(22) Date of filing: 22.10.2001
(51) Int. Cl.: A61K 9/127, A61Q 19/10, A61Q 19/00

(54) **New non-phospholipid lipid vesicles (npLV) and their use in cosmetic, therapeutic and prophylactic applications**
Non-phospholipid Vesikel (npLV) und ihre Verwendung in kosmetischen, therapeutischen und prophylaktischen Anwendungen
Vesicles non-phospholipidiques (npLV) et leur utilisation en cosmetique, therapeutique et preventive

(43) Date of publication of application: 23.04.2003
(73) Proprietor: Viroblock SA, 1288 Genève (CH)
(72) Inventor: Wallach, Donald F.H., 1208 Genève (CH)
(74) Representative: Jelsch, Emmanuel Edwin

(56) References cited:
- WO-A-95/16436
- WO-A-95/16437
- US-A- 5 160 669
- US-A- 5 256 422

## Description

The present invention concerns new lipid vesicles wherein all said lipids are non phospholipid lipids, methods of preparation thereof as well as their use as vehicle particularly in therapeutic applications such as prevention of AIDS.

### DESCRIPTION OF THE PRIOR ART

Liposomes are microscopic lipid vesicles than can carry a volume of water or non-aqueous material and can, in principle, serve in the transport, delivery and function of a vast number of agents, including drugs, enzymes, nucleic acids, dermatological and fuel additives.

Liposomes may be made either from phospholipid amphiphiles, or from non-phospholipid "membrane mimetic" agents. The latter are here called non-phospholipid lipid vesicles, or npLV. All polar lipids, phospholipid and non-phospholipid carry a hydrophilic head group at one end and a hydrophobic residue at the other and therefore possess molecular amphiphily.

Interest in lipid vesicles is intense as attested by more than 500 research publications and a greater number of patents over the past half decade.

However, although lipid vesicles have been hyped for thirty years, there have been very few practical applications.

This is largely because of manufacturing problems arising from an obsession with phospholipids as materials, techniques developed more than three decades ago for phospholipids, as well as needless limitation to costly niche pharmaceutical applications.

Apart from the cost of phospholipids and limited flexibility, phospholipid vesicle technology is beset by many practical problems.

Phospholipids are expensive to purify or synthesize, and their manufacture is difficult, cumbersome, expensive and often dangerous to scale up.

Phospholipid vesicles are labile and for most intended *in vivo* uses require important chemical modifications.

Phospholipid vesicles were seen since more than 60 years ago and defined in the laboratory by Bangham et al. (1965; "Diffusion of univalent ions across the lamellae of swollen phospholipid; J. Mol. Biol. 13; 228-252), who also developed means for the hydration of phospholipid layers dried from organic solvents, to form vesicles.

This technique, developed for academic research continues to dominate most of the field.

Most methods for making phospholipid liposomes involve conversion of lamellar phases into liposomes.
(A) Most commonly, a film of the phospholipid, prepared by deposition from an organic solvent, is hydrated with an aqueous medium, forming lamellar phases.
(B) A further method involves dissolution of the phospholipid in an organic solvent, followed by mixing with an aqueous medium and removal of the solvent.
(C) In a third method, the phospholipids are solubilized by detergents and the detergent concentration is then reduced, by dialysis, below the critical micelle concentration of the detergent, allowing liposomes to form.
(D) In a recent approach, organic solvents have been replaced by super-fluid gases acting as organic solvents, such as CO₂, mixtures thereof with other gaseous hydrocarbons, freon, at appropriate conditions of pressure and temperature. As these are depressurized by injection into aqueous media, phospholipid-bounded bubbles form to lamellar phases yielding liposomes.

The lamellar phases products formed by all of these methods are converted into liposomes of desired sizes using a variety of procedures, including shaking, stirring, extrusion through plastic or ceramic filters of appropriate porosity, brute force cavitation/shearing (e.g. "Microfluidizer", ultrasonic irradiation).

In some applications, the lamellar phase particles or the liposomes may be dried on solid or porous surfaces or lyophilized, for further processing. Important disadvantages continue in terms of application to a pharmaceutical or industrial scale, because of the need to remove organic solvents or detergents to apply drastic procedures, and issues of sizing, and stability.

Non-phospholipid Lipid Vesicles (npLV) are a more recent development. However, the first laboratory demonstrations in the 1970s were followed by research showing that a wide variety of non-phospholipid amphiphiles could form vesicles a circumstance that has led to a bewildering array of papers, mostly pharmacy publications.

It is now clear, however, that npLV can perform nearly all the tasks envisioned for phospholipid vesicles and many tasks that phospholipid vesicles cannot perform.

Currently known amphiphiles forming lipid vesicles include fatty acyl block copolymers, fatty acids, long-chain soaps in the presence of nonionic surfactants, single-tailed ether derivatives of polyglycerol, fatty acyl sucrose esters, sorbitan monoesters, crown ethers, synthetic galactolipids; two-headed ammonium amphiphiles, sonicated double-tailed cationic surfactants, cationic or zwitterionic two-chain amphiphiles involving amino acid residues, diacyl-cysteine, mixtures of single-tailed cationic and anionic surfactants, and ethoxylated perfluorocarbon alcohols.

Despite the l'Oreal group's extensive development of npLV approaches to skin care in the late 1980s researches have continued to use one or other variations or combinations of above methods (A) and (C), creating lamellar phases and then reducing these to liposomes.

They have thus not eliminated the need to use/remove organic solvents or detergents and have not dispensed with the need for expensive processing of extended lamellar phases.

In contrast, the previous procedures of the applicant first transform membrane-forming lipids into "flowable" liquids. The hot mixtures are then hydrated. After hydration, the mixture is cooled, with continued shear-mixing, causing the liposome precursor particles to coalesce into "paucilamellar" vesicles (up to 7 bilayers).

Reciprocating syringes are used for small quantities. For large-scale productions, a continuous flow machine is employed.

The procedures have allowed production of lipid vesicles composed of nonionic amphiphiles and steroids, vesicles carrying various oils and hydrophobic materials, vesicles with alkyds as wall-forming materials vesicles having N,N-dimethylamide derivatives as primary lipid, perfluorocarbon and gas carrying lipid vesicles, hybrid liposomes including phospholipids as wall materials, vesicles carrying functional hemoglobin, lipid vesicles delivering minoxidil to the skin, lipid vesicles acting as or carrying immunological adjuvants, lipid vesicles acting in cell and viral fusion, vesicles delivering antacid and other oral products and other applications.

NpLV of the type made by the procedures of US 5019174, 5160669 and 5019392 were intended to transport apolar material, such as oils, waxes, resins, drugs, nutrients, biocides and perfluorocarbon liquids, avoiding the unfavorable partition of such materials into bilayer phases.

In one procedure of US 5019174, preformed npLVs are mixed at near-ambient temperatures, under low shear conditions with the water-immiscible cargo in the presence of an indifferent surfactant suitable for the emulsification of the cargo lipid.

In the process of US 5160669, the heated, water-immiscible substance is combined with the hot, liquid, amphiphile used to make vesicle membranes. The mixture is injected into the aqueous phase.

"Lipid-coated microbubbles", LCM, FILMIX®, have been proposed to avoid the problems associated with phospholipid liposomes. These are stable gas-in-liquid emulsions formed by mechanical agitation of aqueous lipid suspensions. However, it is unclear how such microbubbles can substitute for lipid vesicles.

The approach earlier introduced by the applicant has not been developed as it might have been.

Its disadvantages include the following :
- The syringe method, designed to test the capacity of various amphiphiles to form npLVs and to encapsulate potential cargo materials, has served this purpose well, but is not suitable for production levels and precision.
- The first single syringe stroke (up to 10 ml), used to inject the hot, liquid lipid mixture into the heated aqueous phase through a 1 mm orifice, yields a micelle mixture which is converted into liposomes of varying sizes by the subsequent syringe strokes.
   Because the amphiphiles, and their viscosities, are very temperature dependent, it is crucial to control the initiating temperature, to regulate the diameter and length of the orifice, as well as the stroke velocity, according to need and to control the temperature of the orifice.
   Too small an orifice would lead to dangerous heating and cavitation and formation of unstable micelles.
   Not all orifice diameters and lengths can be used (and an orifice diameter of 1.4 µm disclosed in US 5626021 does not seem feasible). Optimally no more than 5 ml can be handled per minute.
   Increasing stroke speed or the volume per stroke increases shear, heating and cavitation.
   In any event, particle size will be heterogeneous.
   For nearly all lipids involved, viscosity (hence flow rate) varies sharply with temperature above melting and some viscous lipids cannot be used at all in this apparatus.
- In the continuous flow method streams of liquid lipid and heated aqueous phase at a desired ratio are combined in a small mixing chamber at high shear velocities and the resulting mixture collected.
   The design of the apparatus gives a rough outline of what is required, but there are unanticipated complications.
   Thus, its is necessary that lipid mixture is truly in solution, and that auxiliary molecules, such as cholesterol, are dissolved in the liquid principal amphiphile.
   Furthermore, to provide the correct amphiphile/water ratio, the correct flow ratios at the injection orifices of the mixing chamber must be maintained, otherwise a variable mixture will result. This requires appropriate flow detectors at the orifices of the mixing chamber with rapid feedback to the pumps.
   These must also be able to treat the differing viscosities of the lipid phases.
   As before, temperature control at the output is crucial for this and needs to feed back to the input pumps. Large and heterogeneous particles are probable.
   Finally, with a flow velocity of 0.094-0.472 L lipid/min and 1.9 L aqueous phase/min (5-30 meter/sec) required for the needed liquid shear, the system does not have the flexibility to be scaled down.

### SUMMARY OF THE INVENTION

This invention relates to a new kind of non-phospholipid liposome, less than 1 µm in diameter, built by modular construction, with sterically stabilized bilayers, superior capacity for the transport and delivery of apolar substances for research, industrial, cosmetic and pharmaceutic applications.

The formation of lipid vesicles as proposed in the present invention depends on established physicochemical mechanisms.

Without water, pure amphiphiles can occur as amorphous solids or liquids, depending on temperature.

However, water causes the amphiphile to associate into structures with a hydrophilic surface and a hydrophobic interior, with certain preferred arrays, including micelles, monolayers, single bimolecular layers and lamellar phases.

Which array is assumed depends upon the nature of the amphiphile, its concentration, the proportion of water, the presence of other amphiphiles, presence of salt and other solutes in the aqueous phase and temperature.

Formation of these structures is driven by, not by cohesive forces, but by the "hydrophobic effect", referring to the distortion of hydrogen-bonded water in the vicinity of hydrocarbon chains.

The need for the apolar chains to be excluded from water is apparent from the energy required to hydrate the apolar moieties.

Thus, the free energy expenditure, ΔG, involved in transferring an individual hydrocarbon chain into water is proportional to chain length, approximating -0.6 (3.5n + 1.5m) kcal/mol, wherein n and m, respectively, correspond to the number of CH₃ (methyl) and CH₂ methylene groups (16 kcal/mol of C₁₆ chain).

The greater the chain length, the larger the free energy, and the lower the probability of the chain remaining in water. Adding of a double bond is roughly equivalent to removal of one methylene group. Like chains tend to associate with like chains in a mixed chain system.

The properties of micellar solutions are critical to this invention.

Micellar solutions consist of micelles, clusters of amphiphile molecules (50 to 200 molecules), dispersed in water.

The shapes of these clusters depend on the amphiphile head group and apolar chain, solution conditions, such as solute concentration and electrolytes and temperature.

Many configurations are possible, depending on temperature.

The shapes of micelles depend on the surface area of the amphiphile, the hydrophobic/hydrophilic interface and the curvature of that interface.

Micelles occur in various phases, including long, normal or reversed, rod-shaped/tubular structures, hexagonally packed arrays and body- or face centered, cubically packed spherical particles.

Once the concentration of free amphiphile exceeds a critical value, the Critical Micelle Concentration, (CMC), the concentration of singly dispersed amphiphiles remains essentially constant. An increase in total amphiphile concentration above the CMC implies an increase in the size and/or number of micelles, not the concentration of free amphiphile.

The CMCs of membrane-mimetic amphiphiles are less than 10⁻⁶ M (well below those of ordinary detergents) and generally decline as the number of methyl and methylene carbons in the chain increase.

The systems are very temperature-sensitive and pass through a series of structural transitions as temperature rises or falls. The energy barrier to amphiphile transfer between micelles is high, but even with low CMCs there is slow interchange through the water phase.

Micelles can aggregate into lamellar phases, parallel bimolecular layers separated by thin water films.

Such arrays are stabilized by several interactions, primarily the hydrophobic effect driving apolar moieties out of water, Van Der Waals attractions between ordered amphiphile residues and hydrogen-bonding of amphiphile head groups to the water at the hydrophilic surfaces of each bilayer.

Each bilayer forms a closed membrane, with its apolar residues sequestered away from water. Each layer of interlamellar water is isolated from every other interlamellar water layer and from any internal or external bulk aqueous phase.

At low temperatures, the lipid chains forming the bilayers assume a densely packed, crystalline phase. This is particularly so for long, fully saturated chains with tightly packed head groups.

With increasing temperature, lipid crystalline phases first expand into an ordered phase and then disorder more and more until, at the main transition temperature, Tₘ, the chains become fluid, assuming a liquid crystalline phase. At still higher temperatures the lipid becomes liquid.

### BRIEF DESCRIPTION OF THE DRAWINGS

Figure 1 represents a schematic view of the lipid vesicle according to the invention.

The npLV is formed by the bilayer 1 comprising an outer stabilizing lipid 2. This npLV comprises the aqueous space 3 and the microemulsion particle 4 surrounded by the internal lipid monolayer 5.

Figure 2 represents a schematic view of the method for producing the vesicles according to the invention.

The lipid phase 6 passes through the hydrophobic filter 8 and rejoins the aqueous phase 7 in order to form micelles 9. Cooling 10 permits formation of npLVs 11.

Figure 3 represents the schematic view of filter holder disc which can be used in the method for producing the vesicles according to the invention.

The filter holder 12 comprises four aqueous channels 13 which permit the entry of the aqueous phase 14 in the filter holder disc.

The filter support 15, the filter 16 and the gasket 17 are respectively placed above the filter holder 12.

### DESCRIPTION OF THE INVENTION

### NON PHOSPHOLIPID LIPID VESICLE

This invention relates to a non phospholipid Lipid Vesicle having a diameter of 1 µm or less, wherein said vesicle comprises :
a) at least one external stabilized bilayer comprising :
   - at least one bilayer stabilizing lipid,
   - at least one bilayer structural lipid,
   - at least one bilayer modulating lipid, and
   - at least one bilayer ionogenic lipid;
b) an intravesicular aqueous space; and
c) at least one intravesicular micro-emulsion particle surrounded by an internal lipid monolayer comprising at least one internal surfactant lipid, said intravesicular micro-emulsion particle contains at least one carrier lipid;
and wherein all said lipids are non phospholipid lipids.

Preferably, the non phospholipid Lipid Vesicle of the invention has a diameter comprised between 0.2 µm to 1 µm, preferably between 0.5 µm to 0.8 µm.

### Vesicle Lipid Phase

An encompassing embodiment is that no phospholipid are employed. A further embodiment is that the bilayers are "sterically stabilized".

Yet another embodiment is that the vesicles are provided with microemulsion particles for the transport of oils and materials dissolved therein.

Another important embodiment of this invention is a modular, rather than "one-fits-all" approach to vesicle construction. Preferred molecules, allowing a wide range of interchange according to need, are mentioned below.

Six groups of non-phospholipid lipids are used in the present invention, with the following interlocking functions :
- (1) bilayer structural lipids;
- (2) bilayer stabilizing lipids;
- (3) bilayer-modulating lipids;
- (4) bilayer ionogenic lipids;
- (5) internal surfactants lipids; and
- (6) carrier lipids.

### 1) Bilayer Structural Lipids

The term "Bilayer Structural lipids" refers to lipids which makes up the bulk of the bilayers of the npLV, according to the invention.

Preferably, the bilayer structural lipid represents 50 to 95 mol. %, preferably 70 to 80 mol. % of the lipids composing the external stabilized bilayer.

The term "Mol. %" represents the number of molecules per 100 molecules. For example, 50 mol. % of bilayer structural lipid represents 50 molecules per 100 molecules of the lipids composing the external stabilized bilayer.

Said bilayer structural lipid may have a chain of at least 14 carbon atoms, and form amorphous liquids at temperature between approximately 40 and 100°C.

Preferably, bilayer structural lipids are chosen in the group consisting of C₁₆₋₂₀ alcohol, C₁₆₋₂₀ fatty acid dimethyl amide, C₁₆₋₂₀ fatty acid diethanolamide, C₁₆₋₂₀ glycerol fatty acid monoester, C₁₆₋₁₈ glycerol fatty acid diester, C₁₆₋₂₀ glycol fatty acid ester, C₁₆₋₂₀ glyceryl ethers, di-PEG C₁₄₋₁₈ ether, (PEG)₂₋₁₀ C₁₄₋₂₀ alcohol, (PEG)₃₋₉ glycerol C₁₆₋₁₈ fatty acid ester, C₁₆₋₂₀ sucrose fatty acid ester, "alkyd" monomer, "epoxy" monomer, galactolipid, sorbitan monoester and PEG₄₋₇ fluorocarbon alcohol.

### 2) Bilayer Stabilizing Lipids

The term "Bilayer stabilizing lipids" refers to lipids which protect npLV surfaces against undesirable interactions with other surfaces in order to stabilize vesicles in aqueous liquid at ambient temperature. Bilayer surface stabilizing lipids comprise hydrocarbon chains that can associate with the chains of the bilayer structural lipids.

The water at the surface of hydrated bilayers is bound in a cooperative fashion to polar head groups, with more extensively hydrogen-bonding and higher density than liquid water; it has been referred to as "soft ice".

The stability of water interactions decreases markedly with rising temperature and for this reason bilayers cease to exist above 100°C.

A major problem, particularly for the intravenous administration of phospholipid vesicles, has been the lack of a surface barrier more extensive and substantial than the soft ice layer.

This largely accounts for the short *in vivo* half-life of injected vesicles, due to their phagocytic uptake and interaction with blood vessel linings.

Much effort has gone into reducing the size of liposomes to reduce phagocytosis, but this is less important now because of the development of "sterically stabilized" liposomes to "shield" lipid vesicle surfaces against undesirable interactions with other surfaces, combined with antibody grafting to create targeted, sterically stabilized immunoliposomes.

To make sterically stabilized phospholipid liposomes, polyethyleneglycol (PEG) residues are coupled to the amino groups of phosphatidyl-ethanolamine (PE) in a number of ways, the carbamate derivative being most widely used currently.

The PEGs most commonly used have 30 or 50 residues (M. Wts. 1900 and 500, but larger PEGs can been used).

PEG coupling to cholesterol and other lipids can also been used.

Between PEG, M. Wts of 350-2000, the greater the PEG content and the size of the PEG group, the greater the perturbation of the bilayer in the vicinity of the PEG-phosphatidylethanolamine residue.

There is increasing use of polyethylene glycol derivatization (PEGylation) in general, particularly of material to be injected parenterally partly because of reduced renal filtration, reduced cellular clearance and phagocytosis.

Since the PEG molecules are oriented to the vesicle inside as well as the surface, and are exposed on any intravesicular membranes, some intravesicular aqueous space is taken up by the PEG.

Thus, the larger the PEG residue, the lower the encapsulation of water-soluble molecules by "PEGylated" liposomes.

The coupling techniques currently used for the manufacture of sterically stabilized phospholipid liposomes are needlessly complicated and expensive and can be improved by using the vesicles of this invention.

Such bilayer stabilizing lipid are "off the shelf" at less than 1/1000^{th} of the cost of their phospholipid equivalents.

Bilayer stabilizing lipid represents preferably 0.1 to 10 mol. %, more preferably 0.5 to 5 mol. %, of the lipids composing the external stabilized bilayer.

The preferred sterically stabilizing lipid of this invention may be chosen in the group consisting of Δ5 cholestene (PEG)₂₄ cholesteryl 3β, (PEG)₂₀ C₁₆₋₁₈ alcohol, (PEG)₁₀₋₅₀ C₁₆₋₂₀ alcohol, (PEG)₂₀₋₄₀ C₁₆₋₂₄ alcohol, (PEG)₉ glycerol fatty C₁₆₋₁₈ acid ester, (PEG)₂₀ sorbitan monopalmitate (Tween 40), (PEG)₂₀ sorbitan monostearate (Tween 80), (PEG)₁₀₋₂₀ C₁₆, propoxylated (CH₂CHCH₃ )₂₀₋₅₀ C₁₆₋₂₀ alcohol, C₁₆₋₂₀ aldosamide and C₁₆₋₂₀ hexosamide.

Appropriate matching with the acyl chains of the principal amphiphiles allows the placement, atop the bilayer surfaces, of the desired stabilizing molecules.

PEG lipids of this invention can also be appropriate for the grafting of specific antibodies to the PEG termini as has been described for phospholipid immunoliposomes (See Kirpotin et al.; Sterically stabilized anti-HER2 immunoliposomes; Biochemistry 36, 66-75; and Belsito et al.; Molecular and mesoscopic properties of hydrophilic polymer-grafted phospholipids mixed with phosphatidylcholine in aqueous dispersion; Biophysical J. 78, 1420-1430).

Yet another embodiment is to reduce bulk of the PEG residues and using (PEG)₂ C₁₆ alcohol and thereby using the method of this invention to make "fusion vesicles" for the transfer of molecules to certain cells and other vesicles.

PEG has been widely used to induce fusion between cells but he actions of PEG on membrane fusion are not simply interpreted (see Herrmann, A. et al., 1983, "Effect of polyethylene glycol on the polarity of aqueous solutions and on the structure of vesicle membranes"; Biochim., Biophys. Acta 733, 87-94. MacDonald R. I., 1985, "Membrane fusion due to dehydration by polyethylene glycol, dextran, or sucrose"; Biochemistry, 24, 4058-66. Yamazaki, M et al., 1990, "Deformation and instability in membrane structure of phospholipid vesicles caused by osmophobic association : Mechanical stress model for the mechanism of poly(ethylene glycol)-induced membrane fusion"; Biochemistry 29, 1309-1314).

Yet others (Needham et al. Sanjit 2000, "Liposomes containing active agents", U.S. Patent 6,143,321) have described approaches to graft low molecular weight PEGs to phospholipid micelles and bilayers to prevent fusion of phospholipid bilayers.

In contrast, Martin and Zalipsky (Martin, F. J. and S. Zalipsky, 2001, "Polymer-lipid conjugates for fusion of target membranes," U.S. Patent, 6,224,903) describe methods of phospholipid liposome construction, wherein the outer regions of the vesicles are coated with releasable PEG or related polymers to prevent fusion.

Release of the PEG uncovers viral (or other) fusogenic peptides, thus creating fusogenic vesicles.

### 3) Bilayer Modulating Lipids

The term "Bilayer modulating lipids" refers to lipids acting like cholesterol which modify the physical properties of the bilayer of the npLV, according to the invention. They may include cholesterol and molecules that act like cholesterol in modifying the physical properties of the lipid bilayer. Bilayer modulating lipids are necessary to prevent lateral phase segregation that may cause bilayer leakiness, vesicle aggregation and unwanted vesicle fusion.

Preferably, the bilayer modulating lipid represents 7 to 30 mol. %, more preferably 10 to 25 mol. % of the lipids composing the external stabilized bilayer.

Bilayer modulating lipid may be chosen in the group consisting of preferably cholesterol, cholesterol derivatives such as for example PEG cholesterol, ionogenic cholesterol and surface stabilizing cholesterol, β-sitosterol, ergosterol and phytosterol.

More preferably, bilayer modulating lipids are chosen in the group consisting of cholesterol, cholesterol derivatives and phytosterol.

Sterols such as cholesterol interact with the polar domains of the hemi-bilayers, leaving the chain segments in these regions less free to change configuration than more disordered long chain segments (Scott at al., 1989, Lipid chains and cholesterol : a Monte Carlo Study, Biochemistry 28, 3687-3691; Davies at al., 1990, Effects of cholesterol on configurational disorder in diphosphatidylcholine bilayers, Biochemistry 29, 4368-4373; McIntosh at al., 1992, Structure and cohesive properties of sphingomyelin/diphosphatidylcholine bilayers, Biochemistry 31, 2020-2025 and Smaby et al., 1994, The interfacial interactions of sphingomyelin and diphosphatidylcholine, Biochemistry 33, 9135-9142).

The β-3 hydroxyl group of cholesterol, combined with the otherwise apolar ring of the molecule, is essential.

Through its phase-dependent interaction with acyl chains, cholesterol allows some different chains to intermix without phase segregation and also broadens the range of temperature for the order-to-liquid-crystalline transition, thereby widening the Tₘ.

### 4) Bilayer lonogenic Lipids

The term "Bilayer lonogenic lipids" refers to anionogenic or catiogenic lipids which provides electrostatic charge to the surface of the npLV, according to the invention.

Preferably, the ionogenic lipid represents 0.05 to 5 mol. %, more preferably 0.1 to 3 mol. %, of the lipids composing the external stabilized bilayer.

The ionogenic lipid of the invention may be chosen from anionogenic and/or cationogenic lipid.

The anionogenic lipid may be chosen in the group consisting of cholesteryl 3-phthalate, cholesteryl 3-hemisuccinate, C₁₆₋₂₀ ethoxylated (PEG)₂₋₈ fatty acid, C₁₆₋₂₀ fatty acid, C₁₆₋₁₈ fatty acid sarcosinate and C₁₆₋₁₈ diacyl phosphate. More preferably, anionogenic lipids are chosen in the group consisting of cholesteryl 3-phthalate, cholesteryl 3-hemisuccinate, C₁₆₋₂₀ ethoxylated (PEG)₂₋₈ fatty acid, C₁₆₋₂₀ fatty acid and C₁₆₋₁₈ diacyl phosphate.

The cationogenic lipid may be chosen in the group consisting of cationic/zwitterionic amino acid 2- C_{≥16-}chain amphiphile, C₁₆₋₁₈ betaine, C₁₆ pyridinium bromide, C₁₆-trimethylammonium bromide (CTAB), Δ5 cholestene 3β-O-CO-N-(CH₂)₂-N⁺-(CH₃)₃, Δ5 cholestene 3β-O-CO-(CH2)₂-N⁺(CH₃)₃, dioleoylpropyltrimethyl ammonium bromide (DOTMA), dodecyltrimethyl ammonium bromide (DDTAB) and tetradecyldimethylaminoxide. More preferably, cationogenic lipids are chosen in the group consisting of C₁₆ pyridinium bromide, C₁₆-trimethylammonium bromide (CTAB), Δ5 cholestene 3β-O-CO-N-(CH₂)₂-N⁺-(CH₃)₃, Δ5 cholestene 3β-O-CO-(CH2)₂-N⁺(CH₃)₃, dioleoylpropyltrimethyl ammonium bromide (DOTMA), dodecyltrimethyl ammonium bromide (DDTAB) and tetradecyldimethylaminoxide.

Much higher cationic lipid proportions may be used to form DNA-liposome complexes for transfection.

### 5) and 6) Internal Surfactant Lipids and Carrier Lipids

By "Internal surfactant lipids" we mean lipids which allow the formation and the stabilization of a monolayer intravesicular micro emulsion particle and have chains that do not insert normally into the bilayer of the npLV, according to the invention.

The term "Carrier lipids" refers to lipids which form the micro emulsion particle core of the npLV, according to the invention.

The internal surfactant lipid preferably represents 0.01 to 1.5 mol. %, more preferably 0.05 to 1.0 mol. %, of the lipids composing the external stabilized bilayer.

The internal surfactant lipid may be chosen in the group consisting of C₁₂ -trimethylammoniumbromide, C₉₋₁₀-methylenehexadecanoic acid, C₉₋₁₂ fatty acids, glycerol monolaurate, lauryl dimethylamine oxide, Mono(nonylphenyl) (PEG)_{< 6} ether, propylene glycol monomyristate, sorbitan monolaurate and sucrose monolaurate.

The carrier lipid preferably represents 15 to 150 %, more preferably 30 to 100 %, of the volume of the lipids composing the external stabilized bilayer.

The carrier lipid may be chosen in the group consisting of ethyl butyrate, ethyl caprylate, filtrable mineral oil, low viscosity oil, perfluorocarbon liquid, silicone oil, squalane, trimyristin, triolein and vegetable oil.

Bilayer hydrophobic regions have lower than expected capacity for most apolar molecules because partition of such substances into bilayers is between a structured and an isotropic phase rather than between two isotropic phases.

The partition even of noble gases is 2 to15 fold lower into a bilayer than into a bulk organic phase. The insertion of substantial amounts of non-bilayer, apolar material can disrupt bilayers. Uptake into a bilayer decreases sharply below the Tₘ in the presence of cholesterol even above the Tₘ.

An important embodiment of this invention is to create vesicles with a capacity to stably transport apolar entities such as oils and related water immiscible materials melting below 40°C, as well as a multiplicity of lipophilic drugs.

This embodiment requires that the vesicles be endowed with surfactants that allow formation of an intravesicular droplet(s) of micro emulsion and forming a monolayer containing a microemulsion droplet.

These surfactants are :
- (a) distinct from those forming the bilayers,
- (b) lacking chains capable of forming bilayers,
- (c) having CMCs above those of the membrane forming bilayer,
- (d) having melting points below 40°C,
- (e) used at levels not more than 6 % of the structural bilayer lipid, and
- (f) have appreciable oil solubility.

It is also preferable that the carrier lipids are liquid at room temperature.

The approach used in this invention is illustrated by the following calculation : in a 0.5 µm vesicle with a 0.01 µm bilayer thickness the apolar volume of the outer bilayer would be about 7 x 10⁻³ µm³. For a sterically stabilized vesicle, where 0.01 µm on each side is occupied by PEG molecules and water, there is room for about 6 bilayers, in addition to the outer one.

These will have apolar volumes of about 5.4, 4.2, 2.8, 2.0 and 1.2 x 10⁻³ µm³, a total of about 16 x 10⁻³ µm³, or a total for all vesicle bilayers of about 23 x 10⁻³ µm3.

Since the capacity of a bilayer is less than 1% of an unstructured organic phase, the capacity of the vesicles bilayers would be less than 0.23 x 10⁻³ µm³.

In contrast, a vesicle with an oil droplet 0.20 µm in diameter could have only 2 bilayers in addition to the outer bilayer, giving a total bilayer volume of about 17 x 10⁻³ µm³ with a capacity about 0.17 x 10⁻³ µm³

The volume of the droplet would be about 4.0 x 10⁻³ µm³ and its capacity would be close to 24 times that of the bilayer.

It is, of course possible to have more than one small microemulsion particle within a vesicle.

A critical aspect of this embodiment is the exchange or transfer after cooling between :
- (a) micro emulsion particles and bilayers; and
- (b) micro emulsion particles of one vesicle and those of another vesicle. Point (a) is prohibited by the different chain structures, Tₘs, CMCs and melting points of the bilayers and microemulsion droplets. Point (b) is similarly limited. Although, movements of amphiphiles can occur slowly below the CMC between identical micelles, transfer of the surfactants selected here is impeded by the following barriers :
   (i) the energetics of movement out of the microemulsion droplet into an internal aqueous phase, and *vice versa;*
   (ii) the energetics of movement through one or more bilayers, and *vice versa,* and
   (iii) the energetics of movement into and through the external aqueous phase, and vice versa.

Accordingly, the micro emulsion particles are highly stable.

The intravesicular micro-emulsion particle c) may contain at least one lipophilic active agent such as a cosmetic and/or therapeutic lipophilic compound.

The cosmetic lipophilic compound can be chosen among those usually used in cosmetic applications. Preferably, the cosmetic lipophilic compound are chosen in the group consisting of antioxidants, ceramides, cyclomethicones and other non-viscous silicone fluids, emollients, fragrances, moistening agents, make-up, mineral and biological oils, sterols, tanning agents, vitamin A and derivatives, including retinoids, vitamin E and derivatives.

The therapeutic lipophilic compound are preferably chosen among lipophilic drugs. The lipophilic drug may be chosen in the group consisting of anthralins, cyclosporines and related drugs, lipid-soluble anti cancer drugs such as, for example, taxanes, lipid-soluble antifungals such as, for example, amphotericin-B, fluconazoles, imidazoles, nystatins and tolnaftates, lipid-soluble antibiotics such as, for example, fucidins, gossypols, gossypol derivatives, anti-HIV proteases, gramicidins, nigericins, lipid-soluble androgens, corticosteroids, estrogens and progestins, lipid-soluble anesthetics, such as, for example, alkylphenols, benzocaines, lidocaines, lipid-soluble vitamins, flavors, lipid A and perfluoro octyl bromides.

The lipophilic drug is preferably present at concentrations of 1 ng/ml to 1 mg/ml of carrier lipids.

The lipophilic drug may be chosen in the group consisting of anthralins, cyclosporines and related drugs, lipid-soluble anti cancer drugs such as, for example, taxanes, lipid-soluble antifungals such as, for example, amphotericin-B, fluconazoles, imidazoles, nystatins and tolnaftates, lipid-soluble antibiotics such as, for example, fucidins, gossypols, gossypol derivatives, anti-HIV proteases, gramicidins, nigericins, lipid-soluble androgens, corticosteroids, estrogens and progestins, lipid-soluble anesthetics, such as, for example, alkylphenols, benzocaines, lidocaines, lipid-soluble vitamins, flavors, lipid A and perfluoro octyl bromides.

### Vesicle Aqueous Phase

A large range of aqueous phases is available for this invention, depending many variables, including the desired ionic and osmotic compositions, pH, and heat stability and what is to be encapsulated.

The aqueous space b) may contain at least one hydrophilic active agent such as a cosmetic and/or therapeutic hydrophilic compound selected in the group consisting of antibody, antigen, protein, antiviral lysozyme, antiviral agent MAP30 and GAP31 and analog or derivative thereof, bioengineered molecule, cytokine, drug, gene such as CFTR gene, gene fragment, RNA, DNA, oligonucleotide, peptide hormone and related macromolecule, DNA and RNA-modifying enzyme such as ribonuclease.

It is an embodiment of this invention that compounds which can be encapsulated and transported in phospholipid liposomes can be equally well carried by the vesicles of this invention.

### METHODS FOR THE PREPARATION OF NPLV

The present invention concerns also a method for the preparation of a non phospholipid Lipid Vesicle of the invention, as defined above, comprising the steps of:
a) preparing a hot, dry and amorphous lipid phase composed of at least one bilayer structural lipid, at least one bilayer stabilizing lipid, at least one bilayer-modulating lipid, at least one bilayer ionogenic lipid, at least one internal surfactant lipid and at least one carrier lipid, wherein said lipids are non phospholipid lipids;
b) converting the hot, dry and amorphous lipid phase into sub micron-sized streamlets by passage through at least one hydrophobic filter with 0.3 to 1.0 µm pore sizes directly into a hot aqueous phase; and
c) cooling the sub micron-sized streamlets in order to form the non phospholipid Lipid Vesicle.

The liposomes of the invention are made by a new method that avoids solubilization of lipids by organic solvents or detergents and operates at controlled temperatures up to 75°C.

Before entering the filter, the lipid phase is in the form of an amorphous liquid. The lipid phase may contain 6 groups of non-phospholipids lipids as defined above, in the same proportions.

Upon cooling, when the temperature is reduced to below the Tₘ of the structural lipid, the configurational freedom of these lipids acyl chains is reduced, the rate of *trans to gauche* transitions of the chains drops and the chains tend to assume a straight-chain geometry.

In this geometry there is extensive exposure of hydrophobic residues to water causing condensation of the chains to a liquid-crystalline, quasi-crystalline or crystalline state and fusion of colliding micelles into vesicles.

Within the temperature limits employed in this invention, the still-liquid carrier lipid, with its liquid stabilizing surfactant are trapped within the vesicles.

A specific embodiment of the invention thus requires that the microemulsion lipid continues as a fluid, while the bilayer lipids condense into a crystalline or quasi-crystalline state.

This invention can be implemented on a small, manual small scale (1 to 5 ml/min) using syringes and modified, commercially available filter holders.

For larger scale applications (superior to 2 L/min depending on the lipid), the invention calls for specially constructed filter holders and use of metering pumps to deliver the lipid phase and aqueous phases.

The method allows lipid vesicle production from 10 cL/min to 33 L/min.

Since the hydrophobic microporous filters used in this invention can easily be blocked, the proper application of this invention preferably requires several simple precautions in the handling of the lipid phase.

The lipid or lipid mixture is preferably stored under desiccation to avoid unwanted hydration that will interfere with solution/solubilization.

The lipid phase may be heated by dry heat, above the temperature required to making the lipid mixture "flowable", to uniform optical clarity, without Rayleigh scattering.

Preferably, the lipid phase of step a) is prepared at a temperature comprised between 35 and 100°C, more preferably between 40 and 80°C.

As already stated, cholesterol broadens the Tm, and hence melting range. Also, the working temperature required is not known precisely in all cases.

Furthermore, some crystalline ancillary lipids dissolve (or co-dissolve) more slowly than the principal amphiphile. Dry heat to 75°C for 60 min is usually sufficient. Lower temperatures may be satisfactory and even desirable in some cases.

Thus, polyoxyethylene (2) cetyl ether can be used a little above 40°C and several C₁₄ polyoxyethylene ethers are liquid at room temperature, whereas glycerol monostearate requires more than 70°C.

In any case it is advisable to perform a small-scale, quantitative filtration test to ensure correct hydration ratios.

In some cases, to ensure sterility, it may be useful to heat the lipid phase above 100°C before returning to the target temperature.

Since the aqueous phases do not pass through the hydrophobic filter, many of the restrictions applied to the lipid phases do not apply to the aqueous phases.

The aqueous phase may be at a temperature comprised between 35 and 100°C, more preferably between 40 and 80°C.

Encapsulation, during the vesiculation phase, of hydrophilic molecules superior to 100000 Daltons is likely to be inefficient.

Suspended particles greater than 0.2 to 0.4 µm are clearly not desired, nor are macromolecules that tend to aggregate at the target temperatures.

Most aqueous phases not containing heat-aggregated molecules, can be heat-sterilized before returning to the target temperature.

The cooling in step c) may be made at a temperature comprised between 0 and 40°C, more preferably between 25 and 35°C.

The possible lipid phase/aqueous phase ratios are 1:2-1:5 (33.3%-16.7% lipid).

Higher lipid concentrations may be desirable for high proportions of aqueous phase encapsulation, but run the danger of gel formation in some systems.

The preferred lipid phase/aqueous phase ratios are 1:3.-1:5 (25%-16.7 %).

### HYDROPHOBIC FILTERS

Preferably, the hydrophobic filter used in step b) of the method for the preparation has pore sizes comprised between 0.5 to 0.8 µm.

It is possible to use several hydrophobic filters having different pore sizes in the preparation of a non phospholipid Lipid Vesicle of the invention.

Hydrophobic filters are preferably made of hydrophobic materials such as, for example, polytetafluoroethylene (such as the Fluoropore™ filters made by Millipore Corporation).

Since such filters can contain as much as 5% water in their interstices, they should preferably be desiccated and stored dry and/or washed with hot isopropanol (or other appropriate solvents) and dried.

At the end of a run, before cooling, the filters can be rinsed with hot water, then solvent such as isopropanol, washed and dried for reusing.

### FILTER HOLDERS

One or more hydrophobic filters may be arranged in a filter holder between the lipid phase and the aqueous phase.

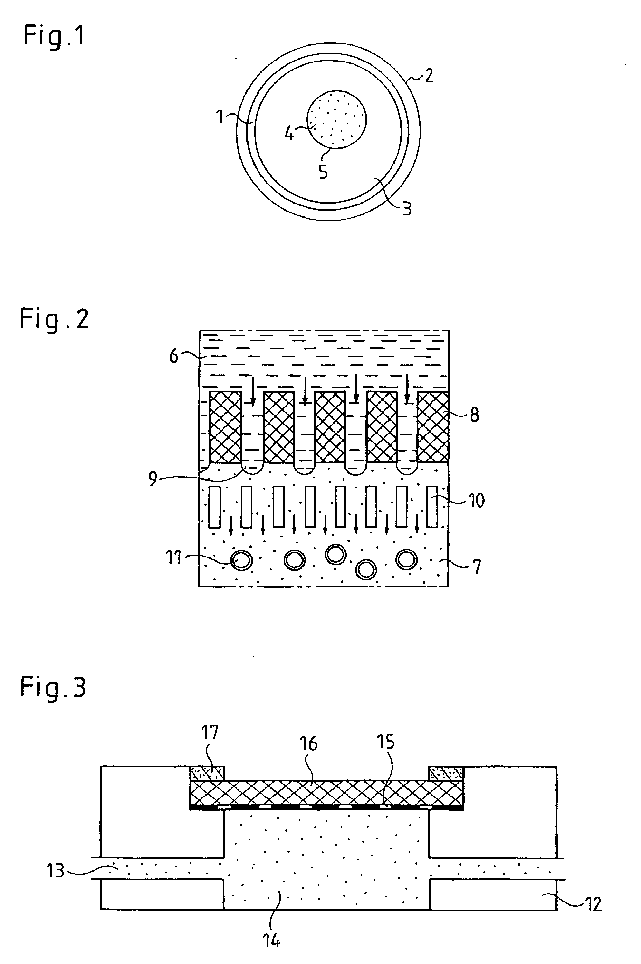

Hydrophobic filters are preferably arranged in filter holder, so that the upper surface of the filter is in contact with the lipid phase and the lower filter surface in contact with the aqueous phase (see Figure 2).

The filter holders are designed to allow the greatest access possible of the stirred and/or flowing, aqueous phase to the lower surface of the hydrophobic principal filter so that the lipid is hydrated immediately after leaving the filter (see Figure 2), avoiding violent shear mixing or vortexing.

This requires a thin rigid (e.g. stainless steel) filter support, with large perforations and minimal blockage of the lower surface of the filter. Many suitable materials are available in the art.

For manual operation (inferior or equal to 5 ml), Swinnex® polypropylene filter holders made by Millipore Corporation can be used.

Equivalent holders may be suitable. In any event, the "male" end of the filter holders must be cut off and the thickness of the filter support milled down to about 1 mm thickness. These filters and its holder are submerged in the stirred, heated aqueous phase.

### PUMP OPERATION

Preferably, the passage through a hydrophobic filter in step b) is enhanced by a pump.

Synchronous, multichannel peristaltic metering pumps are preferred for the transfer of the bulk lipid and aqueous phases.

Examples are the Cole-Palmer Masterflex™ pumps of the LS and IP types (about 10200 L/h and 3900L/h, respectively, for a five channel operation), but other pumping systems may be suitable.

The multiple high-power pumps and high-flow/high-shear should preferably be avoided.

A single drive motor with multiple channels is a preferred means of obtaining fixed, present lipid/aqueous ratios.

For most applications, multiple identical channels are employed, with one channel pumping the lipid phase and 4 channels the aqueous phase.

Many suitable pumps are commercially available.

The lipid and aqueous phases, as well as the pump(s) and delivery lines are maintained at the target temperature.

The bundled, insulated, lipid and aqueous lines are connected by state-of-the art "quick-fit" connectors.

### LARGER SCALE APPLICATIONS

For operations other than the manual scale procedures, specialized filter holders are constructed as an embodiment of the invention.

The holder filters can be manufactured from a variety of materials, ranging from polypropylene to stainless steel.

All are autoclavable or gas-sterilizable.

The holders may be specifically designed for a given each filter diameter, although combinations of small sizes to give the filtration and processing capacity of a single unit can be contemplated. Inserts for large holders to accommodate smaller filters are envisaged.

The central axis of the holder passes through the center of the filter. Each holder has three components, joined to each by clamps, bolts, gaskets and/or other state of the art devices (see Figure 3).

The top compartment is analogous with the Swinnex-type holders in construction, with a single, central lipid-phase inlet and a conical expansion towards the hydrophobic filter.

The bottom of this compartment is made up by the top surface of the filter.

The holder is a disk with a central cutout for the filter and its support and sealing gaskets.

Four aqueous tunnels are bored into the disk, receiving the aqueous phase and directing it on to the bottom of the filter. An insert for smaller filters is shown. A thin, magnetically coupled stirrer lies below the plane of aqueous input. Considering a 293 mm filter with a 70% filter area, the effective filter area will be 1887 cm². At the hypothetical maximum value of about 2000 L/h (see Table 1), or about 33000 ml/min, this gives a linear flow at the filter of about 18 cm/min.

**Table 1 : Some properties of hydrophobic filters**

| **Pore size (µm)** | **Filter diameter (mm)** | **Filtration area (cm²)** | **Flow rate (mL/min)** | | **20% Wt/Wt Vesicle/Suspension (L/h)** | |
|---|---|---|---|---|---|---|
| | | | (PEG)₂C₁₆alc/CH | Olive oil | (PEG)₂C₁₆alc/CH | Olive oil |
| 0.5 | 13 | 0.7 | 5 | 2 | 1.5 | 0.6 |
| | 25 | 3 | 21 | 9 | 6 | 3 |
| | 47 | 14 | 100 | 40 | 30 | 12 |
| | 90 | 48 | 342 | 137 | 103 | 41 |
| | 192 | 220 | 1571 | 528 | 471 | 141 |
| | 293 | 513 | 3664 | 1466 | 1100 | 330 |
| 1.0 | 13 | 0.7 | 10 | 4 | 3 | 1 |
| | 25 | 3 | 42 | 17 | 13 | 5 |
| | 47 | 14 | 200 | 80 | 60 | 24 |
| | 90 | 48 | 686 | 274 | 206 | 82 |
| | 192 | 220 | 3142 | 1257 | 942 | 377 |
| | 293 | 513 | 7329 | 2931 | 2198 | 879 |

Upon emergence from the filter at temperature, the mixtures are a combination of micelles and microemulsion particles, which is converted by a specific embodiment of this invention.

Cooling coils (see Figure 2) in the third, thermo-regulated chamber, thermally insulated from the previous chamber, reduce the temperature of the mixture to below the structural lipid Tₘ (40°C or below).

Assuming a high flow rate of approximately 2000 L/h (see Table 1) for a 75°C mixture, which is 80 % water, possessing nearly all the heat capacity and a 293 mm filter to be cooled to 40°C, would require approximately 1,300 L/h of 15°C water. Many commercial cooling systems can be adapted to this task (e.g. Cole-Palmer Masterflex™ B/T System).

### FURTHER PROCESSING

The present invention concerns equally a method for the preparation of a non phospholipid Lipid Vesicle as defined above, by centrifugation, comprising the steps of:
a) preparing a hot, dry and amorphous lipid phase composed of at least one bilayer structural lipid, at least one bilayer stabilizing lipid, at least one bilayer-modulating lipid, at least one bilayer ionogenic lipid, at least one internal surfactant lipid and at least one carrier lipid, wherein said lipids are non phospholipid lipids;
b) layering the amorphous lipid phase prepared in step a) over an aqueous phase containing dextran for effecting a continuous or discontinuous separation in a bowl rotor in order to convert, by centrifugation, the amorphous lipid phase into sub micron-sized streamlets; and
c) cooling the sub micron-sized streamlets in order to form the non phospholipid Lipid Vesicle.

Preferably, the dextran has a molecular weight comprised between 70000 and 250000 g/mol, more preferably between 100000 and 150000 g/mol.

High Mol. Wt dextrans are used because of their negligible osmotic activities (appropriate polysucrose; Ficoll might be employed, but low molecular weight solutes are not useful).

The centrifugation in step b) is effected preferably between 1000 and 6000 rpm, more preferably between 2000 and 3000 rpm (see Steck et al; A model for the behavior of vesicles in density gradients : Implications for fractionation; Biochim. Biophys. Acta. 203, 385-393). The bowl rotor can be of the type manufactured by the Haemonetics Corporation

Since the density of the internal aqueous phase is about 1.00 and that of the lipid less than 1.00, the vesicles will have a density of less than 1.00, the density barrier will accelerate the rate of migration and the vesicles will migrate to the top of the density barrier.

Any free emulsion particles will migrate more slowly. Upon centrifugation at about 6000 rpm in a bowl rotor the vesicle band will appear at the top of the density barrier. (The volume of the vesicle band, V_{T}, is made up of volume of the hydrated wall lipid, V_{Li,} and the captured water volume, V^{c}).

The vesicle band at the top is taken off as in standard apheresis procedures. The flow of the mixture emerging from the cooling coils is matched, by appropriate detectors and microprocessors, to the flow of the density phase, and the removal of the vesicle volume.

### DEVICE FOR THE PREPARATION OF NPL

The present invention concerns equally a device for the preparation of a non phospholipid Lipid Vesicle (npLV) according to the invention, comprising a filter holder 12 having :
a) at least one aqueous channel 13 permitting the entry of an aqueous phase 14 in said filter holder 12,
b) a filter support 15, and
c) a filter 16.

Preferably, this device comprises a gasket 17.

### COSMETIC USE

The present invention concerns also a cosmetic composition comprising a non phospholipid Lipid Vesicle of the invention.

A non phospholipid Lipid Vesicle as defined above can also be used in cosmetic application, including delivery of antioxidants, ceramides, cyclomethicones and other non-viscous silicone fluids, emollients, fragrances, moistening agents, make-up, mineral and biological oils, sterols, tanning agents, vitamin A and derivatives, including retinoids, vitamin E and derivatives (see Wallach et al., 1995, Some large-scale, non-medical applications of non-phospholipid liposomes, in Nonmedical Application of Liposomes, D. Lasic and Y. Barenholz, editors, CRC press, Boca Raton, FL, pp. 115-125).

### THERAPEUTIC USE

The present invention concerns also a pharmaceutical composition comprising a non phospholipid Lipid Vesicle of the invention. Usually, a such a pharmaceutical composition comprises therapeutically excipients such as anthralins, cyclosporines and related drugs, lipid-soluble anti cancer drugs such as, for example, taxanes, lipid-soluble antifungals such as, for example, amphotericin-B, fluconazoles, imidazoles, nystatins and tolnaftates, lipid-soluble antibiotics such as, for example, fucidins, gossypols, gossypol derivatives, anti-HIV proteases, gramicidins, nigericins, lipid-soluble androgens, corticosteroids, estrogens and progestins, lipid-soluble anesthetics, such as, for example, alkylphenols, benzocaines, lidocaines, lipid-soluble vitamins, flavors, lipid A and perfluoro octyl bromides.

A non phospholipid Lipid Vesicle as defined above can also be used for the preparation of a medication, particularly as a fusogenic vesicle.

An extensive literature deals with the creation of fusogenic phospholipid liposomes for the transfer of DNA and other molecules to target cells.

Very sophisticated techniques have been developed for this purpose using fusogenic viral proteins and peptides incorporated into bilayers (e.g. Martin, F. J. and S. Zalipsky, 2001, "Polymer-lipid conjugates for fusion of target membranes", U.S. Patent, 6,224,903).

The non phospholipid Lipid Vesicle of the invention may be used as a retrovirus virucide for the preparation of a medication for the therapeutic or prophylactic treatment of AIDS.

Outside of cells, HIV and related retroviruses are enclosed by a membrane whose lipids are derived from the membrane lipids of a previous target cell during exit from that cell.

When such viruses enter new target cells, their membranes fuse with those of the new target cell, their RNA is injected into the target cell cytoplasm and is shortly thereafter converted into target cell DNA by reverse transcription. In the sexual transmission of HIV probably no more than 10³ copies of virus per ml.

In the proposed system, the npLV functions as pseudo targets for the virus.

However, following fusion the viral RNA cannot be transcribed, the vesicles lacking the mechanisms, and the viral RNA, and possibly other viral functions, are destroyed by RNAase and other agents included in the intravesicular space.

The antiviral functions are highly directed to free virus or possibly individual semen cells that shed virus before causing a general infection.

The overall structure of the fusogenic vesicle is basically the same as shown in Figure 1.

Lipids and drugs of a such fusogenic vesicle are preferably the following : bilayer modulating lipids can be di-PEG C₁₄₋₁₈ ethers; bilayer modulating lipids may be chosen in the groups consisting of phytosterol and cholesterol; bilayer stabilizing lipids may be chosen in the group consisting of (PEG)₁₀₋₂₀ C₁₆, propoxylated(CH₂CHCH₃)C₁₆ alcohol and C₁₆ aldosamide/hexosamide; lipophilic drugs may be chosen in the group consisting of gramicidin, gossypol, gossypol derivatives and anti-HIV protease and the molecules of the aqueous space may be chosen in the group consisting of antiviral ribonucleases, antiviral lysozyme, antiviral proteins such as MAP30 and GAP31.

The bilayer 1 may be constructed of di-PEG C₁₄₋₁₈ ethers at a 3/1, wt/wt ether/cholesterol ratio. The C₁₆ compound is more effective than the C₁₈ ether, but mixtures of the C₁₄ and the C₁₆ compounds may be more effective still.

The outermost layer 2 is desired to reduce potential fusion with spermatozoa since, without this layer, the vesicles are likely to be highly spermatocidal. However, the component molecules should be limited to chain lengths of C₁₆ and the hydrophilic moieties limited to PEG₂₀ or the equivalent.

The aqueous space 3 may be charged with antiviral ribonucleases, antiviral lysozyme, anti-viral proteins such as MAP30 and GAP31 and related agents at concentrations of 0.1 to 100 µg/ml (see Lee-Huang et al., 1999, "Lysozyme and RNAases as anti-HIV components in beta core preparations of human chorionic gonadotropin", Proc. Natl. Acad. Sci., USA, 96-2678-2681. Schreiber et al., 1999, "The antiviral agents MAP 30 and GAP 31 are not toxic to human spermatozoa and may be useful in preventing the sexual transmission of human immunodefficiency virus type 1"; Fertility and Sterility, 72, 686-90).

The microemulsion particle 5 corresponds to a lipid compartment which can act as a depot for lipid-soluble anti viral agents including gramicidin (Bourinbaiar, A. S. and S. Lee-Huang, 1995, "Rational problems associated with cellular approaches in controlling HIV spread", Adv. Exp. Med. Biol. 374, 71-89), gossypol, gossypol derivatives (Vander Jagt, D. and R. Royer, 1989, U. S. Patent 5,026,726), and anti-HIV proteases.

As produced according to this invention, the concentration of 0.5 µm in diameter vesicles is in the order of 1-2 x 10¹³/cc³.

The range of HIV particles in infected semen is not well known, but probably does not exceed 10³/cc³. An antiviral preparation with a vesicle dilution of 10⁶ (vesicle/virus ratio = 10⁴) can be envisaged.

The non phospholipid Lipid Vesicle of the invention can be also used as a fusogenic vesicle for delivery of drugs and other molecules via the olfactory pathway.

Thus, the non phospholipid Lipid Vesicle of the invention can be used as a fusogenic vesicle for the preparation of a medication able to deliver active agent via the olfactory pathway.

The olfactory system provides a direct, intraneuronal pathway from the nasal epithelium to the central nervous system, by passing the "blood-brain barrier".

Several recent publications provide evidence for such transfer in animals (see Hastings, L. and J.E. Evans, 1991, "Olfactory primary neurons as a route of entry for toxic agents into the CNS"; Neurotoxicology 12, 707-714. Thorne, R.G., Emory, CR, Ala, TA, and WH Frey, 2nd, 1995, "Quantitative analysis of olfactory pathways for drug delivery to the brain"; Brain Res. 18, 692, 272-282. Tjalve, H., Hendrikson, J., Tallkvist, J. , Larsson, B.S. and N.G. Lindquist, 1996, "Uptake of manganese and cadmium into the central nervous system via olfactory pathways in rats"; Pharmacol. Toxicol. 79, 347-356).

Experiments have been conducted to use potentially fusogenic phospholipid liposomes as vehicles for the transfer of the CFTR gene by application to the nasal epithelium of cystic fibrosis patients (see Caplen, N.J., Alton, E.W., Middleton, P.G., Dorin, J.R., Stevenson, B.J., Gao, X., Durham, S.R., Jefferey, P.K.., Hodson, M.E. and C. Coutelle, 1995, "Liposome-mediated gene transfer into the nasal epithelium of patients with cystic fibrosis"; Nature Medicine, 1, 39-56. Boucher, R. C., 1999, "Status of gene therapy for cystic fibrosis lung disease"; J. Clin. Invest, 103, 442-445.).

### EXPERIMENTAL PART

### Example 1

This example is designed to study the effect of using dry heat versus a steam bath (open container):

The lipid mixture was polyoxyethylene (2) cetyl ether/cholesterol, 3/1, wt/wt.

The temperature was 60°C for 60 min.

The filters were Millipore Co. 13 mm, 0.5 or 1.0 µm Fluoropore™ polytetrafluoroethylene filters (with polyethylene backing) in 13 mm Swinnex® polypropylene filter holders.

The "male" end of the filter holders was cut off and the thickness of the filter support milled down to 1 mm thickness.

Five ml of lipid was delivered via 10 ml polypropylene syringes with a pressure of 0.6-0.8 bar at 60°C, avoiding air bubbles. Washing with hot isopropanol, drying and insuring resistance to water flow tested filter integrity.

| **CONDITION** | **EFFECT** |
|---|---|
| Steam bath | Rayleigh scattering 1 µm filter flow slow → stop 0.5 µm filter plugs |
| Dry heat | Optically clear Unhindered flow |

The example shows the adverse effects of water associated with the lipid phase. It indicates that autoclaving of lipid is undesirable.

### Example 2

This example is designed to study the effect of heating to flowability (about 40°C) versus 60°C for 60 min, using dry heat:

The lipid mixture was polyoxyethylene (2) cetyl ether/cholesterol, 3/1, wt/wt.

The temperature was 60°C.

The filters were Millipore Co. 13 mm, 0.5 or 1.0 µm Fluoropore™ polytetrafluoroethylene filters (with polyethylene backing) in 13 mm Swinnex® polypropylene filter holders modified as before.

Five ml of lipid was delivered via 10 ml polypropylene syringes with a pressure of 0.6-0.8 bar at 60°C, avoiding air bubbles. Washing with hot isopropanol, drying and insuring resistance to water flow tested filter integrity.

| **CONDITION** | **EFFECT** |
|---|---|
| To flowability | Rayleigh scattering Filter slow → plug |
| 60°C for 60 min. | Optically clear Unhindered flow |

This example indicates the need to heat to clarity and suggests the need to avoid cooling until after passage through hydrophobic filter.

### Example 3

This example is designed to study the effect of filter temperature.

The lipid mixture was polyoxyethylene (2) cetyl ether/cholesterol, 3/1, wt/wt., heated at 60°C for 1 h.

The filters were Millipore Co. 13 mm, 0.5 or 1.0 µm Fluoropore™ polytetrafluoroethylene filters (with polyethylene backing) in 13 mm Swinnex® polypropylene filter holders modified as before.

Five ml of lipid was delivered via 10 ml polypropylene syringes with a pressure of 0.6-0.8 bar at 60°C, avoiding air bubbles. Filters were tested preheated and at room temperature.

| **CONDITION** | **EFFECT** |
|---|---|
| Preheated filter | Normal flow |
| Room temperature filter | Slow initial flow |

This example indicates that filter temperature influences flow.

As noted above, in protocols, where the lower filter surface is "washed" by the aqueous phase, the filter temperature will be largely determined by the temperature of the aqueous phase.

### Example 4

This example is designed to study an aspect of filter holder construction.

The lipid mixture was polyoxyethylene (2) cetyl ether/cholesterol, 3/1, wt/wt., heated at 60°C for 1 h.

The filters were Millipore Co. 13 mm, 0.5 or 1.0 µm Fluoropore™ polytetrafluoroethylene filters (with polyethylene backing) in 13 mm Swinnex® polypropylene filter holders unmodified or modified as described above.

Five ml of lipid was delivered via 10 ml polypropylene syringes with a pressure of 0.6-0.8 bar at 60°C, avoiding air bubbles. The filter holders were submersed in 20 ml of aqueous phase at 60°C, stirred vigorously, avoiding vortexing and cooled to room temperature. Injection of the lipid produced a milky dispersion. This was evaluated by phase-contrast and polarization microscopy at room temperature within 10 min.

| **CONDITION** | **EFFECT** |
|---|---|
| Unmodified filter holders | Polarization microscopy showed broad size range of budding, multilamellar myelin forms and vesicles. |
| "Trimmed" filter holders | Vesicles, predominantly |

This example suggests that a time interval before hydration allows the formation of a variety of pleomorphic bilamellar structures and that quick hydration favors vesicles.

### Example 5

This example is designed to study effects of filter porosity.

The lipid mixture was polyoxyethylene (2) cetyl ether/cholesterol, 3/1, wt/wt., heated at 60°C for 1 h.

The filters were Millipore Co. 13 mm, 0.5 or 1.0 µm Fluoropore™ polytetrafluoroethylene filters (with polyethylene backing) in 13 mm Swinnex® polypropylene modified filter holders.

Five ml of lipid was delivered via 10 ml polypropylene syringes with a pressure of 0.6-0.8 bar at 60°C, avoiding air bubbles. The filter holders were submersed in 20 ml of aqueous phase at 60°C, stirred vigorously, avoiding vortexing, and cooled to room temperature. Injection of the lipid produced a milky dispersion. This was evaluated by phase-contrast and polarization microscopy at room temperature within 10 min.

| **CONDITION** | **EFFECT** |
|---|---|
| 1.0 µm filters | Flow as in Table 1 Vesicles ≈ 1.0 µm |
| 0.5 µm filters | Flow as in Table 1 Vesicles ≈ 0.5 µm |

This example suggests hydration as the lipid leaves the hydrophobic filter yields vesicles approximating the pore size of the filter.

## Claims

1. A non phospholipid Lipid Vesicle having a diameter of 1 µm or less, wherein said vesicle comprises :
a) at least one external stabilized bilayer comprising :
- at least one bilayer stabilizing lipid,
- at least one bilayer structural lipid,
- at least one bilayer modulating lipid, and
- at least one bilayer ionogenic lipid;
b) an intravesicular aqueous space (3); and
c) at least one intravesicular micro-emulsion particle (4) surrounded by an internal lipid monolayer comprising at least one internal surfactant lipid, said intravesicular micro-emulsion particle contains at least one carrier lipid;
and wherein all said lipids are non phospholipid lipids.

2. A non phospholipid Lipid Vesicle according to claim 1, wherein said vesicle has a diameter comprised between 0.2 µm to 1 µm.

3. A non phospholipid Lipid Vesicle according to anyone of claims 1 to 2, wherein the bilayer structural lipid represents 50 to 95 mol. % of the lipids composing the external stabilized bilayer.

4. A non phospholipid Lipid Vesicle according to anyone of claims 1 to 3, wherein the bilayer structural lipid has a chain of at least 14 carbon atoms, and form amorphous liquids at a temperature comprises between approximately 40 and 100°C.

5. A non phospholipid Lipid Vesicle according to anyone of claims 1 to 4, wherein the bilayer structural lipid is chosen in the group consisting of C₁₆₋₂₀ alcohol, C₁₆₋₂₀ fatty acid dimethyl amide, C₁₆₋₂₀ fatty acid diethanolamide, C₁₆₋₂₀ glycerol fatty acid monoester, C₁₆₋₁₈ glycerol fatty acid diester, C₁₆₋₂₀ glycol fatty acid ester, C₁₆₋₂₀ glyceryl ether, di-PEG C₁₄₋₁₈ ether, (PEG)₂₋₁₀ C₁₄₋₂₀ alcohol, (PEG)₃₋₉ glycerol C₁₆₋₁₈ fatty acid ester, C₁₆₋₂₀ sucrose fatty acid ester, "alkyd" monomer, "epoxy" monomer, galactolipid, sorbitan monoester and PEG₄₋₇ fluorocarbon alcohol.

6. A non phospholipid Lipid Vesicle according to anyone of claims 1 to 5, wherein the bilayer stabilizing lipid represents 0.1 to 10 mol. % of the lipids composing the external stabilized bilayer.

7. A non phospholipid Lipid Vesicle according to anyone of claims 1 to 6, wherein the bilayer stabilizing lipid is chosen in the group consisting of Δ5 cholestene (PEG)₂₄ cholesteryl 3β, (PEG)₂₀ C₁₆₋₁₈ alcohol, (PEG)₁₀₋₅₀ C₁₆₋₂₀ alcohol, (PEG)₂₀₋₄₀ C₁₆₋₂₄ alcohol, (PEG)₉ glycerol fatty C₁₆₋₁₈ acid ester, (PEG)₂₀ sorbitan monopalmitate, (PEG)₂₀ sorbitan monostearate, (PEG)₁₀₋₂₀ C₁₆, propoxylated (CH₂CHCH₃ )₂₀₋₅₀ C₁₆₋₂₀ alcohol, C₁₆₋₂₀ aldosamide and C₁₆₋₂₀ hexosamide.

8. A non-phospholipid Lipid Vesicle according to anyone of claims 1 to 7, wherein the bilayer modulating lipid represents 7 to 30 mol. % of the lipids composing the external stabilized bilayer.

9. A non phospholipid Lipid Vesicle according to anyone of claims 1 to 8, wherein the bilayer modulating lipid is chosen in the group consisting of cholesterol, cholesterol derivative such as PEG cholesterol, ionogenic cholesterol and surface stabilizing cholesterol, β-sitosterol, ergosterol and phytosterol.

10. A non phospholipid Lipid Vesicle according to anyone of claims 1 to 9, wherein the ionogenic lipid represents 0.05 to 5 mol. % of the lipids composing the external stabilized bilayer.

11. A non phospholipid Lipid Vesicle according to anyone of claims 1 to 10, wherein the ionogenic lipid is chosen from anionogenic and/or cationogenic lipid.

12. A non phospholipid Lipid Vesicle according to anyone of claims 1 to 11, wherein the ionogenic lipid is an anionogenic lipid chosen in the group consisting of cholesteryl 3-phthalate, cholesteryl 3-hemisuccinate, C₁₆₋₂₀ ethoxylated (PEG)₂₋₈ fatty acid, C₁₆₋₂₀ fatty acid, C₁₆₋₁₈ fatty acid sarcosinate and C₁₆₋₁₈ diacyl phosphate.

13. A non phospholipid Lipid Vesicle according to anyone of claims 1 to 12, wherein the ionogenic lipid is a cationogenic lipid chosen in the group consisting of cationic/zwitterionic amino acid 2- C_{≥16-}chain amphiphile, C₁₆₋₁₈ betaine, C₁₆ pyridinium bromide, C₁₆-trimethylammonium bromide (CTAB), Δ5 cholestene 3β-O-CO-N-(CH₂)₂-N⁺-(CH₃)₃, Δ5 cholestene 3β-O-CO-(CH2)₂-N⁺(CH₃)₃, dioleoylpropyltrimethyl ammonium bromide (DOTMA), dodecyltrimethyl ammonium bromide (DDTAB) and tetradecyldimethylaminoxide.

14. A non phospholipid Lipid Vesicle according to anyone of claims 1 to 13, wherein the aqueous space b) contains at least one hydrophilic active agent such as a cosmetic and/or therapeutic hydrophilic compound selected in the group consisting of antibody, antigen, protein, antiviral lysozyme, antiviral agent MAP30 and GAP31 and analog or derivative thereof, bioengineered molecule, cytokine, drug, gene such as CFTR gene, gene fragment, RNA, DNA, oligonucleotide, peptide hormone and related macromolecule, DNA and RNA-modifying enzyme such as ribonuclease.

15. A non phospholipid Lipid Vesicle according to anyone of claims 1 to 14, wherein the internal surfactant lipid represents 0.01 to 1.5 mol. % of the lipids composing the external stabilized bilayer.

16. A non phospholipid Lipid Vesicle according to anyone of claims 1 to 15, wherein the internal surfactant lipid is chosen in the group consisting of C₁₂ -trimethylammoniumbromide, C₉₋₁₀-methylenehexadecanoic acid, C₉₋₁₂ fatty acid, glycerol monolaurate, lauryl dimethylamine oxide, Mono(nonylphenyl) (PEG)_{< 6} ether, propylene glycol monomyristate, sorbitan monolaurate and sucrose monolaurate.

17. A non phospholipid Lipid Vesicle according to anyone of claims 1 to 16, wherein the carrier lipid represents 15 to 150 % of the volume of the lipids composing the external stabilized bilayer.

18. A non phospholipid Lipid Vesicle according to anyone of claims 1 to 17, wherein the carrier lipid is chosen in the group consisting of ethyl butyrate, ethyl caprylate, filtrable mineral oil, low viscosity oil, perfluorocarbon liquid, silicone oil, squalane, trimyristin, triolein and vegetable oil.

19. A non phospholipid Lipid Vesicle according to anyone of claims 1 to 18, wherein the intravesicular micro-emulsion particle c) contains at least one lipophilic active agent such as a cosmetic and/or therapeutic lipophilic compound.

20. A non phospholipid Lipid Vesicle according to claim 19, wherein the therapeutic lipophilic compound is a lipophilic drug.

21. A non phospholipid Lipid Vesicle according to claim 20, wherein the lipophilic drug is present at concentrations of 1 ng/ml to 1 mg/ml of carrier lipids.

22. A non phospholipid Lipid Vesicle according to anyone of claims 20 to 21, wherein the lipophilic drug is chosen in the group consisting of anthralin, cyclosporine and related drug, lipid-soluble anti cancer drug such as, for example, taxane, lipid-soluble antifungal such as, for example, amphotericin-B, fluconazole, imidazole, nystatin and tolnaftate, lipid-soluble antibiotic such as, for example, fucidin, gossypol, gossypol derivative, anti-HIV protease, gramicidin, nigericin, lipid-soluble androgen, corticosteroid, estrogen and progestin, lipid-soluble anesthetic, such as, for example, alkylphenol, benzocaine, lidocaine, lipid-soluble vitamin, flavor, lipid A and perfluoro octyl bromide.

23. A non phospholipid Lipid Vesicle according to claim 19, wherein the cosmetic compound is chosen in the group consisting of antioxidant, ceramide, cyclomethicone and other non-viscous silicone fluid, emollient, fragrance, moistening agent, make-up, mineral and biological oil, sterol, tanning agent, vitamin A and derivative, including retinoid, vitamin E and derivative.

24. A method for the preparation of a non phospholipid Lipid Vesicle according to anyone of claims 1 to 23, comprising the steps of:
a) preparing a hot, dry and amorphous lipid phase composed of at least one bilayer structural lipid, at least one bilayer stabilizing lipid, at least one bilayer-modulating lipid, at least one bilayer ionogenic lipid, at least one internal surfactant lipid and at least one carrier lipid, wherein all said lipids are non phospholipid lipids;
b) converting the hot, dry and amorphous lipid phase into sub micron-sized streamlets by passage through at least one hydrophobic filter with 0.3 to 1.0 µm pore sizes directly into a hot aqueous phase; and
c) cooling the sub micron-sized streamlets in order to form the non phospholipid Lipid Vesicle.

25. A method according to claim 24, wherein the lipid phase of step a) is prepared at a temperature comprised between 35 and 100 °C.

26. A method according to anyone of claims 24 to 25, wherein the aqueous phase is at a temperature comprised between 35 and 100 °C.

27. A method according to anyone of claims 24 to 26, wherein the lipid phase/aqueous phase ratio is comprised between 1:2 to 1:5.

28. A method according to anyone of claims 24 to 27, wherein the hydrophobic filter used in step b) has pore sizes comprised between 0.5 to 0.8 µm.

29. A method according to anyone of claims 24 to 28, wherein the hydrophobic filter is made of a hydrophobic material such as polytetrafluoroethylene.

30. A method according to anyone of claims 24 to 29, wherein one or more hydrophobic filters are arranged in a filter holder between the lipid phase and the aqueous phase.

31. A method according to anyone of claims 24 to 30, wherein the passage through a hydrophobic filter in step b) is enhanced by a pump.

32. A method according to anyone of claims 24 to 31, wherein the cooling in step c) is made at a temperature comprised between 0 and 40 °C.

33. A method for the preparation of a non phospholipid Lipid Vesicle according to anyone of claims 1 to 23, comprising the steps of:
a) preparing a hot, dry and amorphous lipid phase composed of at least one bilayer structural lipid, at least one bilayer stabilizing lipid, at least one bilayer-modulating lipid, at least one bilayer ionogenic lipid, at least one internal surfactant lipid and at least one carrier lipid, wherein all said lipids are non phospholipid lipids;
b) layering the amorphous lipid phase prepared in step a) over an aqueous phase containing dextran for effecting a continuous or discontinuous separation in a bowl rotor in order to convert by centrifugation the amorphous lipid phase into sub micron-sized streamlets; and
c) cooling the sub micron-sized streamlets in order to form the non phospholipid Lipid Vesicle.

34. A method according to claim 33, wherein dextran has a molecular weight comprised between 70000 and 250000 g/mol.

35. A method according to anyone of claims 33 to 34, wherein the centrifugation in step b) is effected between 1000 and 6000 rpm.

36. A cosmetic composition comprising a non phospholipid Lipid Vesicle according to anyone of claims 1 to 23.

37. A cosmetic use of a non phospholipid Lipid Vesicle according to anyone of claims 1 to 23.

38. A pharmaceutical composition comprising a non phospholipid Lipid Vesicle according to anyone of claims 1 to 23.

39. An use of a non phospholipid Lipid Vesicle according to anyone of claims 1 to 23 for the preparation of a medication.

40. An use of a non phospholipid Lipid Vesicle according to claim 39 as a fusogenic vesicle for the preparation of a medication.

41. An use of a non phospholipid Lipid Vesicle according to anyone of claims 39 and 40 as a retrovirus virucide for the preparation of a medication for the therapeutic or prophylactic treatment of AIDS.

42. An use of a non phospholipid Lipid Vesicle according to claim 41, wherein the bilayer modulating lipid is di-PEG C₁₄₋₁₈ ether; the bilayer modulating lipid is chosen in the groups consisting of phytosterol and cholesterol; the bilayer stabilizing lipid is chosen in the group consisting of (PEG)₁₀₋₂₀ C₁₆, propoxylated(CH₂CHCH₃)C₁₆ alcohol and C₁₆ aldosamide/hexosamide; the lipophilic drug is chosen in the group consisting of gramicidin, gossypol, gossypol derivative and anti-HIV protease and the active agent of the aqueous space is chosen in the group consisting of antiviral ribonuclease, antiviral lysozyme, antiviral protein such as MAP30 and GAP31.

43. An use of a non phospholipid Lipid Vesicle according to anyone of claims 39 to 40 as a fusogenic vesicle for the preparation of a medication able to deliver active agent via the olfactory pathway.

44. Device for the preparation of a non phospholipid Lipid Vesicle (npLV) according to anyone of claims 1 to 23, comprising a filter holder (12) having :
a) at least one aqueous channel (13) permitting the entry of an aqueous phase (14) in said filter holder (12),
b) a filter support (15), and
c) a filter (16).

## Patentansprüche

1. Ein nichtphospholipides Lipidvesikel mit einem Durchmesser von 1 µm oder weniger, worin das besagte Vesikel umfasst:
a) mindestens eine stabilisierte äussere Doppelschicht, mit:
- mindestens einem die Doppelschicht stabilisierenden Lipid,
- mindestens einem die Doppelschicht strukturierenden Lipid,
- mindestens einem die Doppelschicht modulierenden Lipid, und
- mindestens einem ionogenen Lipid der Doppelschicht;
b) einen intravesikularen wässrigen Hohlraum (3); und
c) mindestens ein intravesikulares Mikroemulsionspartikel (4), das von einer inneren Lipideinzeilschicht umgeben ist, welche mindestens ein inneres oberflächenaktives Lipid umfasst, wobei das besagte intravesikulare Mikroemulsionspartikel mindestens ein Trägerlipid enthält;
und worin alle besagten Lipide nichtphospholipide Lipide sind.

2. Ein nichtphospholipides Lipidvesikel gemäss Anspruch 1, worin das besagte Vesikel einen Durchmesser zwischen 0.2 µm und 1 µm aufweist.

3. Ein nichtphospholipides Lipidvesikel gemäss irgendeinem der Ansprüche 1 bis 2, worin das die Doppelschicht strukturierende Lipid 50 bis 95 Molekularprozent der Lipiden, welche die stabilisierte äussere Doppelschicht bilden, darstellt.

4. Ein nichtphospholipides Lipidvesikel gemäss irgendeinem der Ansprüche 1 bis 3, worin das die Doppelschicht strukturierende Lipid eine Kette von mindestens 14 Kohlenstoffatomen aufweist, und bei einer Temperatur zwischen ca. 40 bis 100°C amorphe Flüssigkeiten bildet.

5. Ein nichtphospholipides Lipidvesikel gemäss irgendeinem der Ansprüche 1 bis 4, worin das die Doppelschicht strukturierende Lipid ausgewählt wird aus der Gruppe bestehend aus C₁₆₋₂₀ Alkohol, C₁₆₋₂₀ Fettsäuredimethylamid, C₁₆₋₂₀ Fettsäurediethanolamid, C₁₆₋₂₀ Glycerylfettsäuremonoester, C₁₆₋₁₈ Glycerylfettsäurediester, C₁₆₋₂₀ Glycolfettsäureester, C₁₆₋₂₀ Glycerylether, di-PEG C₁₄₋₁₈ Ether, (PEG)₂₋₁₀ C₁₄₋₂₀ Alkohol, (PEG) ₃₋₉ Glyceryl C₁₆₋₁₈ Fettsäureester, C₁₆₋₂₀ Sukrosefettsäureester, Alkydmonomer, Epoxidmonomer, Galaktolipid, Sorbitanmonoester und PEG₄₋₇ Fluorokohlenstoffalkohol.

6. Ein nichtphospholipides Lipidvesikel gemäss irgendeinem der Ansprüche 1 bis 5, worin das die Doppelschicht stabilisierende Lipid 0.1 bis 10 Molekularprozent der Lipiden, welche die stabilisierte äussere Doppelschicht bilden, darstellt.

7. Ein nichtphospholipides Lipidvesikel gemäss irgendeinem der Ansprüche 1 bis 6, worin das die Doppelschicht stabilisierende Lipid ausgewählt wird aus der Gruppe bestehend aus Δ5 Cholestene (PEG)₂₄ Cholesteryl 3β, (PEG)₂₀ C₁₆₋₁₈ Alkohol, (PEG)₁₀₋₅₀ C₁₆₋₂₀ Alkohol, (PEG)₂₀₋₄₀ C₁₆₋₂₄ Alkohol, (PEG)₉ Glycerol C₁₆-₁₈ Fettsäureester, (PEG)₂₀ Sorbitanmonopalmitat, (PEG)₂₀ Sorbitanmonostearat, (PEG)₁₆₋₂₀ C₁₆, propoxyliertem (CH₂CHCH₃)₂₀₋₅₀ C₁₆₋₂₀ Alkohol, C₁₆₋₂₀ Aldosamid und C₁₆₋₂₀ Hexosamid.

8. Ein nichtphospholipides Lipidvesikel gemäss irgendeinem der Ansprüche 1 bis 7, worin das die Doppelschicht modulierende Lipid 7 bis 30 Molekularprozent der Lipiden, welche die stabilisierte äussere Doppelschicht bilden, darstellt.

9. Ein nichtphospholipides Lipidvesikel gemäss irgendeinem der Ansprüche 1 bis 8, worin das die Doppelschicht modulierende Lipid ausgewählt wird aus der Gruppe bestehend aus Cholesterin, Cholesterinderivat wie PEG Cholesterin, ionogenem Cholesterin und oberflächenstabilisierendem Cholesterin, β-Sitosterin, Ergosterin und Phytosterin.

10. Ein nichtphospholipides Lipidvesikel gemäss irgendeinem der Ansprüche 1 bis 9, worin das ionogene Lipid 0.05 bis 5 Molekularprozent der Lipiden, welche die stabilisierte äussere Doppelschicht bilden, darstellt.

11. Ein nichtphospholipides Lipidvesikel gemäss irgendeinem der Ansprüche 1 bis 10, worin das ionogene Lipid aus den anionogenen und/oder kationogenen Lipiden ausgewählt wird.

12. Ein nichtphospholipides Lipidvesikel gemäss irgendeinem der Ansprüche 1 bis 11, worin das ionogene Lipid ein anionogenes Lipid ist, ausgewählt aus der Gruppe bestehend aus Cholesteryl 3-Phthalat, Cholesteryl 3-Hemisuccinat, C₁₆₋₂₀ ethoxyliertem (PEG)₂₋₈ Fettsäure, C₁₆₋₂₀ Fettsäure, C₁₆₋₁₈ Fettsäuresarcosinat und C₁₆₋₁₈ Diacylphosphat.

13. Ein nichtphospholipides Lipidvesikel gemäss irgendeinem der Ansprüche 1 bis 12, worin das ionogene Lipid ein kationogenes Lipid ist, ausgewählt aus der Gruppe bestehend aus kationischer/zwiterrionischer amphiphiler Aminosäure 2- C_{≥16}-Kette, C₁₆₋₁₈ Betaine, C₁₆ Pyridiniumbromid, C₁₆ Cetyltrimethylammoniumbromid (CTAB), Δ5 Cholestene 3β-O-CO-N-(CH₂)₂-N⁺-(CH₃)₃, Δ5 Cholestene 3β-O-CO-(CH₂)₂-N⁺-(CH₃)₃, Dioleoylpropyltrimethylammoniumbromid (DOTMA), Dodecyltrimethylammoniumbromid (DDTAB) und Tetradecyldimethylaminoxyd.

14. Ein nichtphospholipides Lipidvesikel gemäss irgendeinem der Ansprüche 1 bis 13, worin der wässrige Hohlraum b) mindestens ein hydrophiles Aktivagens enthält, wie eine hydrophile kosmetische und/oder therapeutische Verbindung, ausgewählt aus der Gruppe bestehend aus Antikörper, Antigen, Protein, antiviralem Lysozym, antiviralem Agens MAP30 und GAP31 und Ähnlichem oder Derivat davon, biotechnisch entwickeltem Molekül, Zytokin, Arzneimittel, Gen wie CFTR-Gen, Genfragment, RNS, DNS, Oligonucleotid, Peptidhormon und artverwandtem Makromolekül, DNS- und RNS-veränderndem Enzym wie Ribonuklease.

15. Ein nichtphospholipides Lipidvesikel gemäss irgendeinem der Ansprüche 1 bis 14, worin das innere oberflächenaktive Lipid 0.01 bis 1.5 Molekularprozent der Lipiden, welche die stabilisierte äussere Doppelschicht bilden, darstellt.

16. Ein nichtphospholipides Lipidvesikel gemäss irgendeinem der Ansprüche 1 bis 15, worin das innere oberflächenaktive Lipid ausgewählt wird aus der Gruppe bestehend aus C₁₂-Trimethylammoniumbromid, C₉₋₁₀-Methylenhexadecanoidsäure, C₉₋₁₂ Fettsäure, Glycerylmonolaurat, Lauryldimethylaminoxyd, Mono(nonylphenyl) (PEG)_{<6} Ether, Propylenglycolmonomyristat, Sorbitanmonolaurat und Sucrosemonolaurat.

17. Ein nichtphospholipides Lipidvesikel gemäss irgendeinem der Ansprüche 1 bis 16, worin das Trägerlipid 15 bis 150 Prozent des Volumens der Lipiden, welche die stabilisierte äussere Doppelschicht bilden, darstellt.

18. Ein nichtphospholipides Lipidvesikel gemäss irgendeinem der Ansprüche 1 bis 17, worin das Trägerlipid gewählt wird aus der Gruppe bestehend aus Ethylbutyrat, Ethylcaprylat, filtrierbarem Mineralöl, niedrigviskosem Öl, Perfluorkohlenstoff-Flüssigkeit, Silikonöl, Squalan, Trimyristin, Triolein und pflanzlichem Öl.

19. Ein nichtphospholipides Lipidvesikel gemäss irgendeinem der Ansprüche 1 bis 18, worin das intravesikulare Mikroemulsionspartikel c) mindestens ein lipophiles Aktivagens enthält, wie eine kosmetische und/oder therapeutische lipophile Verbindung.

20. Ein nichtphospholipides Lipidvesikel gemäss Anspruch 19, worin die lipophile Verbindung ein lipophiles Arzneimittel ist.

21. Ein nichtphospholipides Lipidvesikel gemäss Anspruch 20, worin das lipophile Arzneimittel bei Konzentrationen von 1 ng/ml bis 1 mg/ml Trägerlipide vorhanden ist.

22. Ein nichtphospholipides Lipidvesikel gemäss irgendeinem der Ansprüche 20 bis 21, worin das lipophile Arzneimittel ausgewählt wird aus der Gruppe bestehend aus Anthralin, Cyclosporin und artverwandtem Arzneimittel, fettlöslichem Antikanzerogenarzneimittel wie beispielsweise Taxan, fettlöslichem Fungizid wie beispielsweise Amphotericin-B, Fluconazol, Imidazol, Nystatin und Tolnaftat, fettlöslichem Antibiotikum wie beispielsweise Fucidin, Gossypol, Gossypolderivat, anti-HIV-Protease, Gramizid, Nigerizin, fettlöslichem Androgen, Kortikosteroid, Östrogen und Progestin, fettlöslichem Anätsthetikum wie beispielsweise Alkylphenol, Benzocainum, Lidocainum, fettlöslichem Vitamin, Aromastoff, A-Lipid und Perfluorooctylbromid.

23. Ein nichtphospholipides Lipidvesikel gemäss Anspruch 19, worin die kosmetische Verbindung ausgewählt wird aus der Gruppe bestehend aus Antioxydant, Keramid, Cyclomethiconium und andere nichtviskosen Silikonflüssigkeiten, Weichmacher, Duftstoff, Feuchthaltemittel, Schminkmittel, Mineralöl und biologischem Öl, Sterin, Gerbstoff, Vitamin A und Derivat einschliesslich Retinoid, Vitamin E und Derivat.

24. Verfahren für die Herstellung eines nichtphospholipiden Lipidvesikels gemäss irgendeinem der Ansprüche 1 bis 23, mit den folgenden Schritten:
a) Herstellung einer heissen, trockenen und amorphen Lipidphase mit mindestens einem die Doppelschicht strukturierenden Lipid, mindestens einem die Doppelschicht stabilisierenden Lipid, mindestens einem die Doppelschicht modulierenden Lipid, mindestens einem ionogenen Lipid der Doppelschicht, mindestens einem inneren oberflächenaktiven Lipid und mindestens einem Trägerlipid, worin alle besagten Lipide nichtphospholipide Lipide sind;
b) Umwandlung der heissen, trockenen und amorphen Lipidphase in Ströme im Submikronbereich durch Durchfluss durch mindestens einen hydrophoben Filter mit Porengrössen von 0.3 bis 1.0 µm direkt in eine heisse wässrige Phase; und
c) Kühlung der Submikronströme, um die nichtphospholipiden Lipidvesikel zu bilden.

25. Ein Verfahren gemäss Anspruch 24, worin die Lipidphase des Schrittes a) bei einer zwischen 35 und 100°C liegenden Temperatur aufbereitet wird.

26. Ein Verfahren gemäss irgendeinem der Ansprüche 24 bis 25, worin die wässrige Phase bei einer Temperatur zwischen 35 und 100°C stattfindet.

27. Ein Verfahren gemäss irgendeinem der Ansprüche 24 bis 26, worin das Verhältnis der Lipidphase zur wässrigen Phase zwischen 1:2 bis 1:5 liegt.

28. Ein Verfahren gemäss irgendeinem der Ansprüche 24 bis 27, worin der im Schritt b) verwendete hydrophobe Filter Porengrössen zwischen 0.5 bis 0.8 µm aufweist.

29. Ein Verfahren gemäss irgendeinem der Ansprüche 24 bis 28, worin der hydrophobe Filter aus einem hydrophoben Material wie Polytetrafluorethylen hergestellt wird.

30. Ein Verfahren gemäss irgendeinem der Ansprüche 24 bis 29, worin ein oder mehr hydrophobe Filter zwischen der Lipidphase und der wässrigen Phase in einem Filterträger angeordnet sind.

31. Ein Verfahren gemäss irgendeinem der Ansprüche 24 bis 30, worin der Durchfluss durch einen hydrophoben Filter im Schritt b) durch eine Pumpe gesteigert wird.

32. Ein Verfahren gemäss irgendeinem der Ansprüche 24 bis 31, worin das Abkühlen im Schritt c) bei einer zwischen 0 und 40°C liegenden Temperatur erfolgt.

33. Ein Verfahren für die Herstellung eines nichtphospholipiden Lipidvesikels gemäss irgendeinem der Ansprüche 1 bis 23, mit den folgenden Schritten:
a) Herstellung einer heissen, trockenen und amorphen Lipidphase mit mindestens einem die Doppelschicht strukturierenden Lipid, mindestens einem die Doppelschicht stabilisierenden Lipid, mindestens einem die Doppelschicht modulierenden Lipid, mindestens einem ionogenen Lipid der Doppelschicht, mindestens einem inneren oberflächenaktiven Lipid und mindestens einem Trägerlipid, worin alle besagten Lipide nichtphospholipide Lipide sind;
b) Auflagerung der im Schritt a) aufbereiteten amorphen Lipidphase über eine wässrige, Dextran enthaltende Phase, um eine durchgehende oder diskontinuierliche Trennung in einem Wannenrotor zu erreichen, um durch Zentrifugieren die amorphe Lipidphase in Ströme im Submikronbereich zu konvertieren; und
c) Kühlung der Submikronströme, um die nichtphospholipiden Lipidvesikel zu bilden.

34. Ein Verfahren gemäss Anspruch 33, worin Dextran ein Molekulargewicht zwischen 70000 und 250000 g/mol aufweist.

35. Ein Verfahren gemäss irgendeinem der Ansprüche 33 bis 34, worin das Zentrifugieren im Schritt b) zwischen 1000 und 6000 rpm erfolgt.

36. Eine kosmetische Zusammensetzung mit einem nichtphospholipiden Lipidvesikel gemäss irgendeinem der Ansprüche 1 bis 23.

37. Eine kosmetische Verwendung eines nichtphospholipiden Lipidvesikels gemäss irgendeinem der Ansprüche 1 bis 23.

38. Eine pharmazeutische Zusammensetzung mit einem nichtphospholipiden Lipidvesikel gemäss irgendeinem der Ansprüche 1 bis 23.

39. Die Verwendung eines nichtphospholipiden Lipidvesikels gemäss irgendeinem der Ansprüche 1 bis 23 für die Herstellung eines Arzneimittels.

40. Die Verwendung eines nichtphospholipiden Lipidvesikels gemäss Anspruch 39 als fusogenes Vesikel für die Herstellung eines Arzneimittels.

41. Die Verwendung eines nichtphospholipiden Lipidvesikels gemäss irgendeinem der Ansprüche 39 und 40 als Retrovirusviruzid für die Herstellung eines Arzneimittels für die therapeutische oder prophylaktische Behandlung von AIDS.

42. Die Verwendung eines nichtphospholipiden Lipidvesikels gemäss Anspruch 41, worin das die Doppelschicht strukturiende Lipid di-PEG C₁₄₋₁₈ Ether ist; das die Doppelschicht modulierende Lipid ausgewählt wird aus der Gruppe bestehend aus Phytosterol und Cholesterol; das die Doppelschicht stabilisierende Lipid ausgewählt wird aus der Gruppe bestehend aus (PEG)₁₀₋₂₀ C₁₆, propoxyliertem (CH₂CHCH₃)C₁₆ Alkohol und C₁₆ Aldosamid/Hexosamid; das lipophile Arzneimittel ausgewählt wird aus der Gruppe bestehend aus Gramizidin, Gossypol, Gossypolderivat und anti-HIV-Protease, und das Aktivagens der wässrigen Phase ausgewählt wird aus der Gruppe bestehend aus antiviraler Ribonuklease, antiviralem Lysozym, antiviralem Protein MAP30 und GAP31.

43. Die Verwendung eines nichtphospholipiden Lipidvesikels gemäss irgendeinem der Ansprüche 39 bis 40 als fusogenes Vesikel für die Herstellung eines Arzneimittels, welches das Aktivagens über den oflaktorischen Weg zu liefern vermag.

44. Vorrichtung für die Herstellung eines nichtphospholipiden Lipidvesikels (npLV) gemäss irgendeinem der Ansprüche 1 bis 23, umfassend einen Filterträger (12) mit:
a) mindestens einem wässrigen Kanal (13), der das Eintreten einer wässrigen Phase (14) in den besagten Filterträger (12) erlaubt,
b) einem Filterträger (15), und
c) einem Filter (16).

## Revendications

1. Une vésicule lipidique non-phospholipidique possédant un diamètre de 1 µm ou moins, dans laquelle ladite vésicule comprend:
a) au moins une bicouche externe stabilisée comprenant:
- au moins un lipide stabilisant la bicouche,
- au moins un lipide structurant la bicouche,
- au moins un lipide modulant la bicouche, et
- au moins un lipide ionogène de la bicouche;
b) un espace aqueux intravésiculaire (3); et
c) au moins une particule de micro-émulsion intravésiculaire (4) entourée par une monocouche lipidique interne comprenant au moins un lipide surfactant interne, ladite particule de micro-émulsion intravésiculaire contenant au moins un lipide porteur;
et dans laquelle tous lesdits lipides sont des lipides non-phospholipidiques.

2. Une vésicule lipidique non-phospholipidique selon la revendication 1, dans laquelle ladite vésicule possède un diamètre compris entre 0.2 µm à 1 µm.

3. Une vésicule lipidique non-phospholipidique selon n'importe laquelle des revendications 1 à 2, dans laquelle le lipide structurant la bicouche représente 50 à 95 en pourcentage moléculaire des lipides composant la bicouche externe stabilisée.

4. Une vésicule lipidique non-phospholipidique selon n'importe laquelle des revendications 1 à 3, dans laquelle le lipide structurant la bicouche possède une chaîne d'au moins 14 atomes de carbone, et forme des liquides amorphes à une température comprise entre approximativement 40 à 100°C.

5. Une vésicule lipidique non-phospholipidique selon n'importe laquelle des revendications 1 à 4, dans laquelle le lipide structurant la bicouche est choisi dans le groupe constitué d'alcool C₁₆₋₂₀, amide diméthylique d'acide gras C₁₆₋₂₀, diéthanolamide d'acide gras C₁₆₋₂₀, monoester glycérolique d'acide gras C₁₆₋₂₀, diester glycérolique d'acide gras C_{16-18,} ester glycolique d'acide gras C₁₆₋₂₀, éther glycérylique C₁₆₋₂₀, éther di-PEG C₁₄₋₁₈, alcool (PEG)₂₋₁₀ C₁₄₋₂₀, ester d'acide gras C₁₆₋₁₈ de glycérol (PEG)₃₋₉, ester d'acide gras de sucrose C₁₆₋₂₀, monomère alkyde, monomère époxy, galactolipide, monoester de sorbitan et alcool fluorocarburé PEG₄₋₇.

6. Une vésicule lipidique non-phospholipidique selon n'importe laquelle des revendications 1 à 5, dans laquelle le lipide stabilisant la bicouche représente 0.1 à 10 en pourcentage moléculaire des lipides composant la bicouche externe stabilisée.

7. Une vésicule lipidique non-phospholipidique selon n'importe laquelle des revendications 1 à 6, dans laquelle le lipide stabilisant la bicouche est choisi dans le groupe constitué de Δ5 cholestène (PEG)₂₄ cholestéryl 3β, alcool C₁₆₋₁₈ (PEG)₂₀, alcool C₁₆₋₂₀ (PEG)₁₀₋₅₀, alcool C₁₆₋₂₄ (PEG)₂₀₋₄₀, ester d'acide gras C₁₆₋₁₈ de glycérol (PEG)₉, monopalmitat de sorbitan (PEG)₂₀, monostéarat de sorbitan (PEG)₂₀, alcool propoxylé C₁₆₋₂₀ (PEG)₁₆₋₂₀ C₁₆, (CH₂CHCH₃)₂₀₋₅₀, aldosamide C₁₆₋₂₀ et hexosamide C₁₆₋₂₀.

8. Une vésicule lipidique non-phospholipidique selon n'importe laquelle des revendications 1 à 7, dans laquelle le lipide modulant la bicouche représente 7 à 30 en pourcentage moléculaire des lipides composant la bicouche externe stabilisée.

9. Une vésicule lipidique non-phospholipidique selon n'importe laquelle des revendications 1 à 8, dans laquelle le lipide modulant la bicouche est choisi dans le groupe constitué de cholestérol, dérivé de cholestérol tel que cholestérol PEG, cholestérol ionogène et cholestérol stabilisateur de surface, β-sitostérol, ergostérol et phytostérol.

10. Une vésicule lipidique non-phospholipidique selon n'importe laquelle des revendications 1 à 9, dans laquelle le lipide ionogène représente 0.05 à 5 en pourcentage moléculaire des lipides composant la bicouche externe stabilisée.

11. Une vésicule lipidique non-phospholipidique selon n'importe laquelle des revendications 1 à 10, dans laquelle le lipide ionogène est choisi parmi les lipides anionogènes et/ou cationogènes.

12. Une vésicule lipidique non-phospholipidique selon n'importe laquelle des revendications 1 à 11, dans laquelle le lipide ionogène est un lipide anionogène choisi dans le groupe constitué de cholestéryl 3-phthalate, cholestéryl 3-hemisuccinate, acide gras éthoxylé C₁₆₋₂₀ (PEG)₂₋₈, acide gras C₁₆₋₂₀, sarcosinat d'acide gras C₁₆₋₁₈ et phosphate di-acylique C₁₆₋₁₈.

13. Une vésicule lipidique non-phospholipidique selon n'importe laquelle des revendications 1 à 12, dans laquelle le lipide ionogène est un lipide cationogène choisi dans le groupe constitué d'acide aminé cationique/zwitterionique amphiphile 2- chaîne C_{≥16}, bétaïne C₁₆₋₁₈, bromure de pyridinium C₁₆, bromure de cétyltriméthylammonium C₁₆ (CTAB), Δ5 cholestène 3pO-CO-N-(CH₂)₂-N⁺-(CH₃)₃, Δ5 cholestène 3β-O-CO-(CH₂)₂-N⁺-(CH₃)₃, bromure de dioleoylpropyltriméthylammonium (DOTMA), bromure de dodécyltriméthylammonium (DDTAB) et aminoxide diméthylique de tétradécyl.

14. Une vésicule lipidique non-phospholipidique selon n'importe laquelle des revendications 1 à 13, dans laquelle l'espace aqueux b) contient au moins un agent actif hydrophilique tel qu'un composé hydrophilique cosmétique et/ou thérapeutique choisi dans le groupe constitué d'anticorps, antigène, protéine, lysozyme antiviral, agent antiviral MAP30 et GAP31 et similaire ou leurs dérivés, molécule issue des biotechnologies, cytokine, médicament, gène tel que le gène CFTR (régulateur de la conductance transmembranaire de la mucoviscidose), un fragment de gène, ARN, ADN, oligonucléotide, hormone peptidique et macromolécule apparentée, enzyme modifiant l'ADN et l'ARN telle que la ribonucléase.

15. Une vésicule lipidique non-phospholipidique selon n'importe laquelle des revendications 1 à 14, dans laquelle le lipide surfactant interne représente 0.01 à 1.5 en pourcentage moléculaire des lipides composant la bicouche externe stabilisée.

16. Une vésicule lipidique non-phospholipidique selon n'importe laquelle des revendications 1 à 15, dans laquelle le lipide surfactant interne est choisi dans le groupe constitué de bromure de triméthylammonium C₁₂, acide de méthylène hexadécanoïque C₉₋₁₀, acide gras C₉₋₁₂, monolaurat glycérolique, aminoxide diméthylique de lauryl, éther mono(nonylphénylique) (PEG)_{<6}, monomyristat glycolique de propylène, monolaurat de sorbitan et monolaurat de sucrose.

17. Une vésicule lipidique non-phospholipidique selon n'importe laquelle des revendications 1 à 16, dans laquelle le lipide porteur représente 15 à 150 pourcent du volume des lipides composant la bicouche externe stabilisée.

18. Une vésicule lipidique non-phospholipidique selon n'importe laquelle des revendications 1 à 17, dans laquelle le lipide porteur est choisi dans le groupe constitué de butyrate d'éthyle, caprylate d'éthyle, huile minérale filtrable, huile à basse viscosité, liquide perfluorocarburé, huile de silicone, squalane, trimyristine, trioléine et huile végétale.

19. Une vésicule lipidique non-phospholipidique selon n'importe laquelle des revendications 1 à 18, dans laquelle la particule de micro-émulsion intravésiculaire c) contient au moins un agent actif lipophilique tel qu'un composé lipophilique cosmétique et/ou thérapeutique.

20. Une vésicule lipidique non-phospholipidique selon la revendication 19, dans laquelle le composé lipophilique thérapeutique est un médicament lipophilique.

21. Une vésicule lipidique non-phospholipidique selon la revendication 20, dans laquelle le médicament lipophilique est présent à des concentrations de 1 ng/ml à 1 mg/ml de lipides porteurs.

22. Une vésicule lipidique non-phospholipidique selon n'importe laquelle des revendications 20 à 21, dans laquelle le médicament lipophilique est choisi dans le groupe constitué d'anthraline, cyclosporine et médicament apparenté, médicament anticancéreux liposoluble tel que par exemple taxane, fongicide liposoluble tel que par exemple amphotéricine-B, fluconazole, imidazole, nystatine et tolnaftate, antibiotique liposoluble tel que par exemple fucidine, gossypol, dérivé de gossypol, protéase anti-HIV, gamicidine, nigéricine, androgène liposoluble, corticostéroïde, oestrogène et progestine, anesthésique liposoluble tel que par exemple phénol alkyl, benzocaïne, lidocaïne, vitamine liposoluble, arôme, lipide A et bromure octyl perfluoré.

23. Une vésicule lipidique non-phospholipidique selon la revendication 19, dans laquelle le composé cosmétique est choisi dans le groupe constitué d'antioxydant, céramide, cyclométhicone et autre fluide de silicone non visqueux, émollient, parfum, agent humectant, maquillant, huile minérale et biologique, stérol, matière tannante, vitamine A et dérivé y compris rétinoïde, vitamine E et dérivé.

24. Un procédé pour la préparation d'une vésicule lipidique non-phospholipidique selon n'importe laquelle des revendications 1 à 23, comprenant les étapes de:
a) préparation d'une phase lipide chaude, sèche et amorphe, composée d'au moins un lipide structurant la bicouche, au moins un lipide stabilisant la bicouche, au moins un lipide modulant la bicouche, au moins un lipide ionogène de la bicouche, au moins un lipide surfactant interne et au moins un lipide porteur, dans laquelle tous lesdits lipides sont des lipides non-phospholipidiques;
b) conversion de la phase lipide chaude, sèche et amorphe en flux de taille submicronique par passage au travers d'au moins un filtre hydrophobe avec des pores d'une taille de 0.3 à 1.0 µm directement en une phase aqueuse chaude; et
c) refroidissement des flux de taille submicronique pour former la vésicule lipidique non-phospholipidique.

25. Un procédé selon la revendication 24, dans lequel la phase lipide de l'étape a) est préparée à une température comprise entre 35 et 100°C.

26. Un procédé selon n'importe laquelle des revendications 24 à 25, dans lequel la phase aqueuse se déroule à une température comprise entre 35 et 100°C.

27. Un procédé selon n'importe laquelle des revendications 24 à 26, dans lequel le rapport de la phase lipide à la phase aqueuse est compris entre 1:2 à 1:5.

28. Un procédé selon n'importe laquelle des revendications 24 à 27, dans lequel le filtre hydrophobe utilisé dans l'étape b) possède des pores d'une taille entre 0.5 à 0.8 µm.

29. Un procédé selon n'importe laquelle des revendications 24 à 28, dans lequel le filtre hydrophobe est réalisé en un matériau hydrophobe tel que le polytétrafluoréthylène.

30. Un procédé selon n'importe laquelle des revendications 24 à 29, dans lequel un ou plusieurs filtres hydrophobes sont disposés dans un porte-filtre entre la phase lipide et la phase aqueuse.

31. Un procédé selon n'importe laquelle des revendications 24 à 30, dans lequel le passage au travers d'un filtre hydrophobe dans l'étape b) est amélioré par une pompe.

32. Un procédé selon n'importe laquelle des revendications 24 à 31, dans lequel le refroidissement de l'étape c) est effectué à une température comprise entre 0 et 40°C.

33. Un procédé pour la préparation d'une vésicule lipidique non-phospholipidique selon n'importe laquelle des revendications 1 à 23, comprenant les étapes de:
a) préparation d'une phase lipide chaude, sèche et amorphe, composée d'au moins un lipide structurant la bicouche, au moins un lipide stabilisant la bicouche, au moins un lipide modulant la bicouche, au moins un lipide ionogène de la bicouche, au moins un lipide surfactant interne et au moins un lipide porteur, dans laquelle tous lesdits lipides sont des lipides non-phospholipidiques;
b) dépose en couche de la phase lipide amorphe préparée dans l'étape a) sur une phase aqueuse contenant de la dextrane pour effectuer une séparation continue ou discontinue dans un rotor à cuve afin de convertir par centrifugation la phase liquide amorphe en flux de taille submicronique; et
c) refroidissement des flux de taille submicronique pour former la vésicule lipidique non-phospholipidique.

34. Un procédé selon la revendication 33, dans lequel la dextrane possède un poids moléculaire compris entre 70000 et 250000 g/mol.

35. Un procédé selon n'importe laquelle des revendications 33 à 34, dans lequel la centrifugation de l'étape b) s'effectue entre 1000 et 6000 rpm.

36. Une composition cosmétique comprenant une vésicule lipidique non-phospholipidique selon n'importe laquelle des revendications 1 à 23.

37. L'utilisation cosmétique d'une vésicule lipidique non-phospholipidique selon n'importe laquelle des revendications 1 à 23.

38. Une composition pharmaceutique comprenant une vésicule lipidique non-phospholipidique selon n'importe laquelle des revendications 1 à 23.

39. L'utilisation d'une vésicule lipidique non-phospholipidique selon n'importe laquelle des revendications 1 à 23 pour la préparation d'un médicament.

40. L'utilisation d'une vésicule lipidique non-phospholipidique selon la revendication 39 comme vésicule fusogénique pour la préparation d'un médicament.

41. L'utilisation d'une vésicule lipidique non-phospholipidique selon n'importe laquelle des revendications 39 et 40 comme virucide de rétrovirus pour la préparation d'un médicament pour le traitement thérapeutique ou prophylactique du SIDA.

42. L'utilisation d'une vésicule lipidique non-phospholipidique selon la revendication 41, dans laquelle le lipide structurant la bicouche est de l'éther di-PEG C₁₄₋₁₈; le lipide modulant la bicouche est choisi dans le groupe constitué de phytostérol et cholestérol; le lipide stabilisant la bicouche est choisi dans le groupe constitué d'alcool propoxylé (CH₂CHCH₃) C₁₆ (PEG)₁₀₋₂₀, et aldosamide/hexosamide C₁₆; le médicament lipophilique est choisi dans le groupe constitué de gramicidine, gossypol, dérivé de gossypol et protéase anti-VIH, et l'agent actif de la phase aqueuse est choisi dans le groupe consistant en ribonucléase antivirale, lysozyme antiviral, protéine antivirale telle que MAP30 et GAP31.

43. L'utilisation d'une vésicule lipide non-phospholipidique selon n'importe laquelle des revendications 39 à 40 comme vésicule fusogénique pour la préparation d'un médicament capable de fournir l'agent actif par le biais de la voie olfactive.

44. Dispositif pour la préparation d'une vésicule lipidique non-phospholipidique (nPLV) selon n'importe laquelle des revendications 1 à 23, comprenant un porte-filtre (12) possédant:
a) au moins un canal aqueux (13) permettant à la phase aqueuse (14) d'entrer dans ledit porte-filtre (12),
b) un support de filtre (15), et
c) un filtre (16).
